# EUROPEAN PATENT APPLICATION

(11) **EP 3 443 976 A1**
(43) Date of publication of application: **20.02.2019**
(21) Application number: 18180179.6
(22) Date of filing: 30.06.2008
(51) Int. Cl.: A61K 38/00

(54) **TISSUE SPECIFIC PEPTIDE CONJUGATES AND METHODS**

(30) Priority: 29.06.2007 US 937725 P
(62) Divisional of application: 08768848.7
(71) Applicant: Sarepta Therapeutics, Inc., Cambridge, MA 02142 (US)
(72) Inventor: MOULTON, Hong, M., Corvallis, OR 97330 (US); IVERSEN, Patrick, L., Grand Junction, CO 81507 (US)
(74) Representative: D Young & Co LLP

(57) **Abstract**

Cell-penetrating peptides useful for targeting a therapeutic compound to a selected mammalian tissue, methods for their identification, methods of forming conjugate compounds containing such peptides, and conjugates formed thereby are disclosed. The cell-penetrating peptides are 8 to 30 amino acid residues in length and consist of subsequences selected from the group consisting of RXR, RX, RB, and RBR; where R is arginine, B is β-alanine, and each X is independently -C(O)-(CHR¹)ₙ-NH-, where n is 4-6 and each R¹ is independently H or methyl, such that at most two R¹'s are methyl. In one embodiment, X is a 6-aminohexanoic acid residue.

## Description

### FIELD OF THE INVENTION

The invention relates to cell-penetrating peptides useful for tissue-specific biodistribution of conjugates containing the peptides, and to methods of selecting such peptides for use in selected tissues.

### REFERENCES

Abes, S., H.M. Moulton et al. (2006). "Vectorization of morpholino oligomers by the (R-Ahx-R)4 peptide allows efficient splicing correction in the absence of endosomolytic agents." J Control Release 116(3): 304-13.
Arap, W. et al. (2004). "Human and mouse targeting peptides identified by phage display." U.S. Appn. Pubn. No. 20040170955.
Behlke, M. A. (2006). "Progress towards in vivo use of siRNAs." Mol Ther 13(4): 644-70.
Alter, J., F. Lou et al. (2006). "Systemic delivery of morpholino oligonucleotide restores dystrophin expression bodywide and improves dystrophic pathology." Nat Med 12(2): 175-7.
Chen, C.P., L.R. Zhang et al. (2003). "A concise method for the preparation of peptide and arginine-rich peptide-conjugated antisense oligonucleotide." Bioconjug Chem 14(3): 532-8.
Gebski, B.L., C.J. Mann et al. (2003). "Morpholino antisense oligonucleotide induced dystrophin exon 23 skipping in mdx mouse muscle." Hum Mol Genet 12(15): 1801-11.
Jearawiriyapaisarn, Moulton et al. (2008). "Sustained Dystrophin Expression Induced by Peptide-conjugated Morpholino Oligomers in the Muscles of mdx Mice." Mol Therapy, June 10, 2008 (advance online publication).
Kang, S.H., M.J. Cho et al. (1998). "Up-regulation of luciferase gene expression with antisense oligonucleotides: implications and applications in functional assay development." Biochemistry 37(18): 6235-9.
Kolonin, M.G., J. Sun et al. (2006). "Synchronous selection of homing peptides for multiple tissues by in vivo phage display." FASEB J 20(7): 979-81.
Meade, B.R. and S.F. Dowdy (2007). "Exogenous siRNA delivery using peptide transduction domains/cell penetrating peptides." Adv Drug Deliv Rev 59(2-3): 134-40.
Richard, J.P., K. Melikov et al. (2003). "Cell-penetrating peptides. A reevaluation of the mechanism of cellular uptake." J Biol Chem 278(1): 585-90.
Rothbard, J.B., E. Kreider et al. (2002). "Arginine-rich molecular transporters for drug delivery: role of backbone spacing in cellular uptake." J Med Chem 45(17): 3612-8.
Samoylova, T.I. and B.F. Smith (1999). "Elucidation of muscle-binding peptides by phage display screening." Muscle Nerve 22(4): 460-6.
Sazani, P., F. Gemignani et al. (2002). "Systemically delivered antisense oligomers upregulate gene expression in mouse tissues." Nat Biotechnol 20(12): 1228-33.
Sontheimer, E. J. (2005). "Assembly and function of RNA silencing complexes." Nat Rev Mol Cell Biol 6(2): 127-38.
Vodyanoy, V. et al. (2003). "Ligand sensor devices and uses thereof." U.S. Appn. Pubn. No. 20030640466.
Wu, R.P., D.S. Youngblood et al. (2007). "Cell-penetrating peptides as transporters for morpholino oligomers: effects of amino acid composition on intracellular delivery and cytotoxicity." Nucleic Acids Res. 35(15):5182-91. (Epub 2007 Aug 1.)
Youngblood, D.S., S.A. Hatlevig et al. (2007). "Stability of cell-penetrating peptide-morpholino oligomer conjugates in human serum and in cells." Bioconjug Chem 18(1): 50-60.

### BACKGROUND OF THE INVENTION

The practical utility of many drugs having potentially useful biological activity is often hindered by problems in delivering such drugs to their targets. The delivery of drugs and other compounds into cells generally occurs from an aqueous cellular environment and entails penetration of a lipophilic cell membrane to gain cell entry.

Oligonucleotides and their analogs are one class of potentially useful drugs whose practical utility has been impeded due to insufficient cellular uptake, and it has been proposed heretofore to enhance uptake of oligonucleotides through conjugation of arginine-rich peptides containing non-a amino acids (see, for example, Chen, Zhang *et al.* 2003; Abes, Moulton *et al*. 2006; Youngblood, Hatlevig *et al.* 2007; and Wu *et al.* 2007). The use of arginine-rich peptides has been reported for the transport of therapeutic drugs, more generally (see, for example, Rothbard, Kreider *et al*. 2002).

Studies by the inventors and others (Chen, Zhang *et al*. 2003; Abes, Moulton *et al.* 2006; Youngblood, Hatlevig *et al*. 2007) have established that incorporation of unnatural amino acids can confer enhanced stability to peptide carriers and enhanced antisense activity to conjugated oligomers, and therefore improve the potential of CPPs (cell penetrating peptides) to deliver therapeutic macromolecules. Despite these advances, there remains a need for CPPs with improved characteristics, and in particular, optimized cell uptake in a selected target tissue.

### SUMMARY OF THE INVENTION

The invention includes, in one aspect, a method for identifying a cell-penetrating peptide useful for targeting a therapeutic compound, typically an oligomeric antisense compound, to a selected mammalian tissue. The method includes the steps of:
(a) forming a library of peptide conjugates composed of
   (i) a plurality of different peptides, each 8 to 30 amino acid residues in length, and preferably 8 to 20 amino acid residues in length, and consisting of subsequences selected from the group consisting of RXR, RX, RB, and RBR; where R is arginine (preferably L-arginine), B is β-alanine, and each X is independently -C(O)-(CHR¹)ₙ-NH-, where n is 4-6 and each R' is independently H or methyl, such that at most two R¹'s are methyl, and
   (ii) covalently coupled to each peptide via an X, B, or XB linkage, a marker compound whose concentration can be assayed in the cells of the selected tissue;
(b) administering each peptide conjugate to a mammalian subject;
(c) assaying the level of the marker compound in cells of the selected tissue, after a period sufficient for localization of the administered peptide conjugate in the selected tissue of the mammalian subject; and
(d) selecting a cell-penetrating peptide useful for targeting a therapeutic compound to the selected mammalian tissue, based on its ability to produce highest or near-highest levels of marker compound, relative to other peptides in the conjugate library, in the selected tissue.

The peptides in the library may include at least 8 peptides selected from the group having sequences identified by SEQ ID NOs: 6-27. In one embodiment, each X residue in the peptide is 6-aminohexanoic acid, the peptide contains at least three X residues, and it comprises a combination of (RXR) and (RBR) subsequences. In another embodiment, each X residue is 6-aminohexanoic acid, the peptide contains at least three X residues, and it comprises a combination of (RX) and (RB) subsequences.

The peptide is typically linked to the marker compound at one terminus, preferably the N-terminus, via a linkage consisting of one or two amino acid residues selected from the group consisting of 6-aminohexanoic acid, 5-aminopentanoic acid, 7-aminoheptanoic acid and β-alanine (such linkages being embodiments of X, B, and XB as described above).

The marker compound in the library conjugates may be a fluorescent marker, where the assaying step includes examining cells from the selected tissue for the presence of internalized fluorescent marker. Preferably, the marker compound in the library of conjugates is an antisense oligomer, which may also be fluorescently labeled. In one embodiment, the antisense oligomer is effective to produce exon skipping or correct aberrant splicing in a selected cellular protein or reporter gene, where the assaying step includes examining the protein products produced by cells of the selected tissue for the presence of the selected cellular protein in a truncated form indicating said exon skipping or splice-correction.

Such an antisense oligonucleotide marker compound may be a phosphorodiamidate morpholino oligonucleotide, and further, a phosphorodiamidate morpholino oligonucleotide containing between about 20-50% positively charged backbone linkages.

The invention also provides specific tissue-selective peptides having a structure as recited above, such as peptides having a sequence selected from SEQ ID NOs: 14-27 below, and in particular peptides having a sequence selected from SEQ ID NOs: 19-27. Other classes of preferred peptides include those in which each X residue in the peptide is 6-aminohexanoic acid, the peptide contains at least three X residues, and it comprises a combination of (RXR) and (RBR) subsequences; and those in which each X residue is 6-aminohexanoic acid, the peptide contains at least three X residues, and it comprises a combination of (RX) and (RB) subsequences. In one embodiment, the peptide has the sequence identified as SEQ ID NO: 19.

In another aspect, the invention includes a method of preparing a therapeutic conjugate for use in treating a disease condition associated with a selected tissue in a mammalian subject, comprising the steps of: (a) identifying a cell-penetrating peptide for the selected tissue, selected by the method disclosed above, (b) selecting a therapeutic compound which is effective against the disease condition when localized in cells of the selected tissue, and (c) conjugating the therapeutic compound to one terminus of the selected cell-penetrating peptide. Preferably, the therapeutic compound is an antisense oligomer, and more preferably a PMO as defined herein.

The method of preparing the conjugate may also include conjugation of a homing peptide which is selective for the selected tissue to another terminus of the cell-penetrating peptide, to form a conjugate of the form: , cell penetrating peptide - homing peptide - therapeutic compound, or, alternatively and preferably, of the form: homing peptide - cell penetrating peptide - therapeutic compound.

In another aspect, the invention provides a peptide-antisense conjugate for treating a given disease condition, comprising a phosphorodiamidate morpholino antisense oligomer directed against a gene whose expression is associated with the given disease condition in a specific target tissue, and covalently linked thereto, via a linkage X, B, or XB, a cell-penetrating peptide that comprises 8 to 20 amino acid residues and consists of a combination of subsequences (RXR) and (RBR), or a combination of subsequences (RX) and (RB), where R is arginine, which may include D-arginine; B is β-alanine; and each X is independently a neutral linear amino acid -C(O)-(CH₂)ₙ-NH-, where n is 4-6, where the cell-penetrating peptide selectively localizes the antisense oligomer in the target tissue. Preferably, n is 5, such that each X is a 6-aminohexanoic acid residue. In further preferred embodiments, the phosphorodiamidate morpholino oligomer contains between about 20-50% positively charged backbone linkages, as described further below.

In the peptide-oligomer conjugates described herein, the conjugated terminus is preferably the N-terminus of the peptide.

Antisense oligomers for treating given disease conditions, typically by inhibiting gene expression or modulating gene splicing, can be designed according to methodologies known in the art, and exemplary sequences are provided herein.

Selected embodiments of peptide-oligomer conjugates of the invention include:
a peptide conjugate compound for use in treating prostate cancer in a mammalian subject, wherein the peptide has the sequence identified as SEQ ID NO: 23, and the antisense oligomer is targeted against human androgen receptor protein;
a peptide conjugate compound for use in treating polycystic kidney disease in a mammalian subject, wherein the peptide has a sequence selected from the group consisting of SEQ ID NOs: 13, 14, 21 and 27, and the antisense oligomer is targeted against human c-myc protein;
a peptide conjugate compound for enhancing stem cell proliferation and survival in peripheral blood, wherein the peptide has a sequence selected from the group consisting of SEQ ID NOs: 10, 14, 19, and 27, and the antisense oligomer is targeted against human TGF-β;
a peptide conjugate compound for use in treating cardiac restenosis, wherein the peptide has a sequence selected from the group consisting of SEQ ID NOs: 19 and 21, and the antisense oligomer is targeted against human c-myc;
a peptide conjugate compound for use in treating a respiratory viral infection, wherein the peptide has the sequence identified by SEQ ID NO: 10, and the antisense oligomer is targeted against influenza A virus or respiratory syncytial virus;
a peptide conjugate compound for use in treating a respiratory bacterial infection, wherein the peptide has a sequence selected from the group identified by SEQ ID NOs: 13, 14, and 19, and the antisense oligomer is targeted against a bacterial 16S rRNA;
a peptide conjugate compound for use in metabolic redirection of a xenobiotic compound normally metabolized in the liver, wherein the peptide has a sequence selected from the group consisting of SEQ ID NOS: 19, 23, 24 and 25; and the antisense oligomer is targeted against a liver P450 enzyme;
a peptide conjugate compound for use in treating viral hepatitis, wherein the peptide has a sequence selected from the group consisting of SEQ ID NOS: 19, 23, 24, and 25; and the antisense oligomer is targeted against hepatitis C virus or hepatitis B virus;
a peptide conjugate compound for use in treating an inflammatory condition in a mammalian subject, wherein the peptide has a sequence selected from the group consisting of SEQ ID NOS: 19, 23, 24, and 25, and the antisense oligomer is effective to induce expression of a soluble TNF-α receptor;
a peptide conjugate compound for use in treating an immune condition in a mammalian subject, wherein the peptide has the sequence represented by SEQ ID NO: 27, and the antisense oligomer is effective to suppress expression of IL-10, CTLA-4, or cFLIP in leukocytes;
a peptide conjugate compound for use in treating loss of skeletal muscle mass in a human subject, wherein the peptide has a sequence selected from the group consisting of SEQ ID NOs: 6, 13, 19, and 20, and the antisense oligomer is targeted against human myostatin; and
a peptide conjugate compound for use in treating Duchenne muscular dystrophy, wherein the peptide has a sequence selected from the group consisting of SEQ ID NOs: 6, 13, 19, and 20, and the antisense oligomer is effective to produce exon skipping in the human dystrophin protein, to restore partial activity of the dystrophin protein. In preferred embodiments of this conjugate, the peptide has the sequence identified as SEQ ID NO: 19, and the antisense oligomer has a sequence selected from the group consisting of SEQ ID NOs: 34 and 49.

In general, the peptide-oligomer conjugate may further comprise a homing peptide which is selective for a selected mammalian tissue, i.e. the same tissue being targeted by the cell-penetrating peptide. The conjugate may be of the form cell penetrating peptide - homing peptide - antisense oligomer, or, more preferably, of the form homing peptide - cell penetrating peptide - antisense oligomer. For example, a peptide conjugate compound for use in treating Duchenne muscular dystrophy, as described above, can further comprise a homing peptide which is selective for muscle tissue, such as the peptide having the sequence identified as SEQ ID NO: 51, conjugated to the cell-penetrating peptide. Exemplary conjugates of this type include those represented herein as CP06062-MSP-PMO (cell penetrating peptide - homing peptide - antisense oligomer) and as MSP-CP06062-PMO (homing peptide - cell penetrating peptide - antisense oligomer) (see appended Sequence Table).

In a related aspect, the invention provides an improvement in a method for preparing an antisense composition for treating Duchenne muscular dystrophy or a muscle-wasting disease in a mammalian subject by preparing an antisense oligomer effective to suppress splice-variant truncations in expressed dystrophin protein, or effective to suppress myostatin expression in muscle tissue, respectively, when administered to a mammalian subject, where the improvement comprises conjugating to the oligomer a cell-penetrating peptide having the sequence identified as SEQ ID NO: 19. The improvement may further include conjugating a muscle-homing peptide, such as SEQ ID NO: 51-55, to the oligomer and cell-penetrating peptide, to form a homing peptide - cell penetrating peptide - antisense oligomer composition.

Also provided is an improvement in a method for preparing an antisense composition for treating an inflammatory condition in a mammalian subject by preparing an antisense oligomer effective to induce expression of a soluble TNF-α receptor, when administered to a mammalian subject (e.g. SEQ ID NO: 33), where the improvement comprises conjugating to the oligomer a cell-penetrating peptide having the sequence selected from the group consisting of SEQ ID NOS: 19, 23, 24, and 25. The improvement may further include conjugating a liver-homing peptide, such as SEQ ID NO: 76, to the oligomer and cell-penetrating peptide, to form a homing peptide - cell penetrating peptide - antisense oligomer composition.

Further provided is an improvement in a method for preparing an antisense composition for treating an immune condition in a mammalian subject by preparing an antisense oligomer effective to suppress expression of IL-10, CTLA-4, or cFLIP in leukocytes, when administered to a mammalian subject, where the improvement comprises conjugating to the oligomer a cell-penetrating peptide having the sequence identified as SEQ ID NO: 27. The improvement may further include conjugating a leukocyte-homing peptide to the oligomer and cell-penetrating peptide, to form a homing peptide - cell penetrating peptide - antisense oligomer composition.

More generally, the invention provides a drug-peptide conjugate for treating a given disease condition, comprising a therapeutic compound or drug whose action is directed against a specific target tissue, and covalently linked thereto, via a linkage X, B, or XB, a cell-penetrating peptide that comprises 8 to 20 amino acid residues and consists of a combination of subsequences (RXR) and (RBR), or a combination of subsequences (RX) and (RB), where R is arginine; B is β-alanine; and each X is independently a neutral linear amino acid -C(O)-(CH₂)ₙ-NH-, where n is 4-6, and is preferably 5; where the cell-penetrating peptide selectively localizes the drug in the target tissue.

As described above, the drug-peptide conjugate may further comprise a homing peptide which is selective for a selected mammalian tissue, i.e. the same tissue being targeted by the cell-penetrating peptide. The conjugate may be of the form cell penetrating peptide - homing peptide - drug, or, more preferably, of the form homing peptide - cell penetrating peptide - drug.

Selected embodiments of peptide-drug conjugates of the invention include:
a conjugate for use in treating breast cancer in a mammalian subject, wherein the cell-penetrating peptide is selected from the group consisting of SEQ ID NOs: 6, 14, 22, and 27, and the therapeutic compound is selected from the group consisting of methotrexate, cyclophosphamide, doxorubicin, 5-fluorouracil, epirubicin, and Herceptin®;
a conjugate for use in treating ovarian or prostate cancer in a mammalian subject, wherein the cell-penetrating peptide has the sequence identified as SEQ ID NO: 23, and the therapeutic compound is selected from the group consisting of (i) an antibody specific against prostate stem cell antigen, for the treatment of prostate cancer, and (ii) taxol, topotecan doxorubicin, Herceptin® and pertuzamab, for the treatment of ovarian cancer;
a conjugate for use in treating cancer of the kidney in a mammalian subject, wherein the cell-penetrating peptide is selected from the group consisting of SEQ ID NOs: 13, 14, 21 and 27, and the therapeutic compound is selected from the group consisting of gemcitabine and capecitabine;
a conjugate for use in treating restenosis, wherein the cell-penetrating peptide is selected from the group consisting of SEQ ID NOs: 19 and 27, and the therapeutic compound is selected from the group consisting of rapamycin and rapamycin analogs having anti-restenosis activity;
a conjugate for use in treating lung cancer in a mammalian subject, wherein the cell-penetrating peptide is selected from the group consisting of SEQ ID NOS: 11, 14 and 19, and the therapeutic compound is selected from the group consisting of cisplatin, carboplatin, paclitaxel, and docetaxel;
a conjugate for use in treating liver cancer in a mammalian subject, wherein the cell-penetrating peptide is selected from the group consisting of SEQ ID NOS: 19, 23, 24 and 25, and the therapeutic compound is selected from the group consisting of doxorubicin, 5-fluorouracil, and methotrexate.

Also included is a conjugate in which the therapeutic compound is a siRNA. Such a conjugate may further include, or be used in conjunction with, a double stranded RNA binding compound to which the siRNA is noncovalently bound.

These and other objects and features of the invention will become more fully apparent when the following detailed description of the invention is read in conjunction with the accompanying drawings.

### BRIEF DESCRIPTION OF THE FIGURES

Figures 1A-C show exemplary structures of a phosphorodiamidate-linked morpholino oligomer (PMO), a peptide-conjugated PMO (PPMO), and a peptide-conjugated PMO having cationic intersubunit linkages (PPMO+), respectively. (Though multiple cationic linkage types are illustrated in Fig. 1C, a PMO+ or PPMO+ oligomer will typically include just one type of cationic linkage.)
Figures 2A-B show the cellular uptake of conjugates of various cell penetrating peptides (CPPs) with carboxyfluorescein-labeled morpholino oligomers (PMOF) in pLuc705 cells.
Figures 3A-D show the nuclear antisense activity of carrier peptide-PMO conjugates in the presence or absence of 10% serum (A-C) or in the presence of up to 60% serum (D).
Figure 4 shows the nuclear antisense activity of carrier peptide-PMO conjugates as a function of the number and position of 6-aminohexanoic acid (Ahx) residues in the peptides.
   The peptides 0, 2, 3a, 3b, 3c, 3d, 4a, 4b, 4c, 5 and 8, corresponding to the number of X residues in the peptide, are shown in Table 1 as SEQ ID NOs: 14, 20, 22, 19, 21, 25, 24, 23, 26, 11 and 3, respectively.
Figures 5A-F show the relative toxicity of carrier peptide-PMO conjugates, as measured by MTT assay.
Figures 6A-D show the relative toxicity of carrier peptide-PMO conjugates as measured by PI exclusion (A-C) and hemolysis (D) assays.
Figures 7A-P show the splice-correction activity in various organs from EGFP-654 transgenic mice treated with various EGFP-654-targeted cell penetrating peptide-PMO conjugates (SEQ ID NOs: 2, 6, 11, 13, 14 and 19-27) as measured in diaphragm (FIG. 7A), mammalian gland (FIG. 7B), ovary and prostate (FIG. 7C), brain (FIG. 7D), kidney (FIG. 7E), bone marrow (FIG. 7F), colon (FIG. 7G), muscle (FIG. 7H), skin (FIG. 7I), spleen (FIG. 7J), stomach (FIG. 7K), thymus (FIG. 7L), heart (FIG. 7M), lungs (FIG. 7N), small intestine (FIG 7O), and liver (FIG 7P).
Figure 8 shows the effect of conjugating an antisense oligomer with a muscle-specific cell penetrating peptide (SEQ ID NO: 19; referred to herein as peptide "B" and also designated CP06062) in combination with a muscle specific homing peptide (MSP), as measured by restoration of full-length dystrophin in the MDX mouse model.

### DETAILED DESCRIPTION

### I. Definitions

The terms below, as used herein, have the following meanings, unless indicated otherwise:
The terms "cell penetrating peptide" or "CPP" are used interchangeably and refer to cationic cell penetrating peptides, also called transport peptides, carrier peptides, or peptide transduction domains. The peptides, as shown herein, have the capability of inducing cell penetration within 100% of cells of a given cell culture population and allow macromolecular translocation within multiple tissues *in vivo* upon systemic administration.

The terms "antisense oligomer" or "antisense compound" are used interchangeably and refer to a sequence of cyclic subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid:oligomer heteroduplex within the target sequence. The cyclic subunits are based on ribose or another pentose sugar or, in a preferred embodiment, a morpholino group (see description of morpholino oligomers below). The oligomer may have exact or near sequence complementarity to the target sequence; variations in sequence near the termini of an oligomer are generally preferaly to variations in the interior.

Such an antisense oligomer is generally designed to block or inhibit translation of mRNA or to inhibit natural pre-mRNA splice processing, and may be said to be "directed to" or "targeted against" a target sequence with which it hybridizes. The target sequence is typically a region including an ATG start codon of an mRNA, or a splice site of a pre-processed mRNA. The target sequence for a splice site may include an mRNA sequence having its 5' end 1 to about 25 base pairs downstream of a normal splice acceptor junction in a preprocessed mRNA. Other nucleic acids such as genomic DNA, rRNA (e.g. in bacteria), or sequences required for replication of viruses may also be targeted. An oligomer is more generally said to be "targeted against" a biologically relevant target, such as a protein, virus, or bacteria, when it is targeted against the nucleic acid of the target in the manner described above.

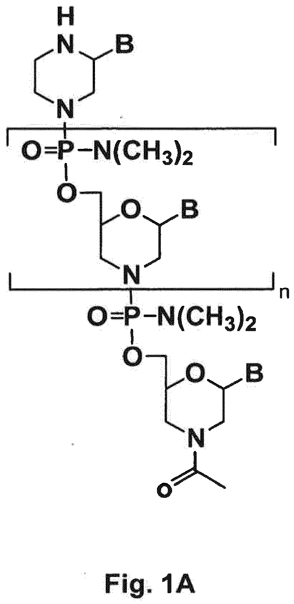

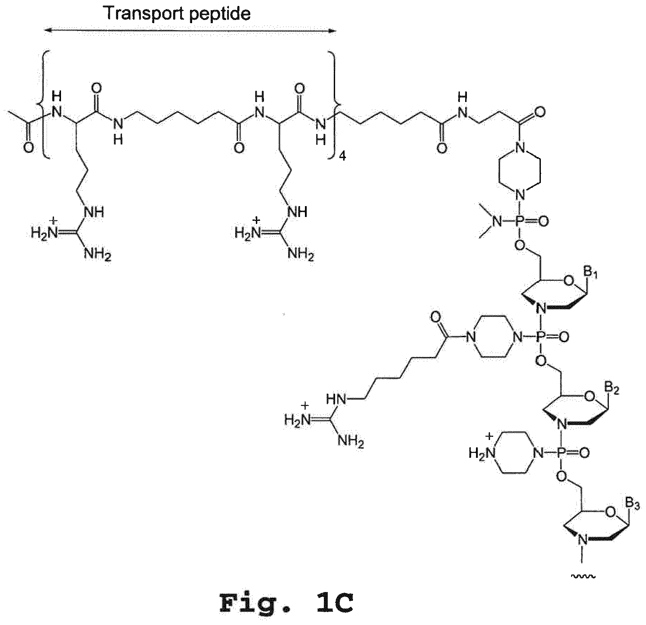

The terms "morpholino oligomer" or "PMO" (phosphoramidate- or phosphorodiamidate morpholino oligomer) refer to an oligonucleotide analog composed of morpholino subunit structures, where (i) the structures are linked together by phosphorus-containing linkages, one to three atoms long, preferably two atoms long, and preferably uncharged or cationic, joining the morpholino nitrogen of one subunit to a 5' exocyclic carbon of an adjacent subunit, and (ii) each morpholino ring bears a purine or pyrimidine base-pairing moiety effective to bind, by base specific hydrogen bonding, to a base in a polynucleotide. See, for example, the structure in Figure 1A, which shows a preferred phosphorodiamidate linkage type. Variations can be made to this linkage as long as they do not interfere with binding or activity. For example, the oxygen attached to phosphorus may be substituted with sulfur (thiophosphorodiamidate). The 5' oxygen may be substituted with amino or lower alkyl substituted amino. The pendant nitrogen attached to phosphorus may be unsubstituted, monosubstituted, or disubstituted with (optionally substituted) lower alkyl. See also the discussion of cationic linkages below. The purine or pyrimidine base pairing moiety is typically adenine, cytosine, guanine, uracil, thymine or inosine. The synthesis, structures, and binding characteristics of morpholino oligomers are detailed in U.S. Patent Nos. 5,698,685, 5,217,866, 5,142,047, 5,034,506, 5,166,315, 5,521,063, and 5,506,337, and PCT Pubn. No. WO 2008036127 (cationic linkages).

An "amino acid subunit" or "amino acid residue" can refer to an α-amino acid residue (-CO-CHR-NH-) or a β- or other amino acid residue (*e*.*g*.-CO-(CH₂)ₙCHR-NH-), where R is a side chain (which may include hydrogen) and n is 1 to 6, preferably 1 to 4.

The term "naturally occurring amino acid" refers to an amino acid present in proteins found in nature. The term "non-natural amino acids" refers to those amino acids not present in proteins found in nature, examples include beta-alanine (β-Ala), 6-aminohexanoic acid (Ahx) and 6-aminopentanoic acid.

A "marker compound" refers to a detectable compound attached to a transport peptide for evaluation of transport of the resulting conjugate into a cell. The compound may be visually or spectrophotometrically detected, e.g. a fluorescent compound or fluorescently labeled compound, which may include a fluorescently labeled oligomer. Preferably, the marker compound is a labeled or unlabeled antisense oligomer. In this case, detection of transport involves detection of a product resulting from modulation of splicing and/or transcription of a nucleic acid by an antisense oligomeric compound. Exemplary methods, such as a splice correction assay or exon skipping assay, are described in Materials and Methods below.

An "effective amount" or "therapeutically effective amount" refers to an amount of therapeutic compound, such as an antisense oligomer, administered to a mammalian subject, either as a single dose or as part of a series of doses, which is effective to produce a desired therapeutic effect. For an antisense oligomer, this effect is typically brought about by inhibiting translation or natural splice-processing of a selected target sequence.

"Treatment" of an individual (e.g. a mammal, such as a human) or a cell is any type of intervention used in an attempt to alter the natural course of the individual or cell. Treatment includes, but is not limited to, administration of a pharmaceutical composition, and may be performed either prophylactically or subsequent to the initiation of a pathologic event or contact with an etiologic agent.

### II. Structural Features of Transport Peptides

In general, a transport peptide as described herein is 8 to 30 amino acid residues in length and consists of subsequences selected from the group consisting of RXR, RX, RB, and RBR; where R is arginine (which may include D-arginine, represented in the sequences herein by r), B is β-alanine, and each X is independently -C(O)-(CHR¹)ₙ-NH-, where n is 4-6 and each R¹ is independently H or methyl, such that at most two R¹'s are methyl. Preferably, each R¹ is hydrogen.

In selected embodiments, the peptide contains at least three X residues and comprises a combination of (RXR) and (RBR) subsequences. In other embodiments, the peptide contains at least three X residues and comprises a combination of (RX) and (RB) subsequences. Preferably, the peptide is 8 to 25, and more preferably 8 to 20, amino acid residues in length.

The variable n is preferably 4 or 5, and more preferably 5, e.g. as in 6-aminohexanoic acid. Unless otherwise indicated, X in peptide sequences represented herein is a 6-aminohexanoic acid residue.

Table 1 below shows the sequences of various transport peptides that were evaluated in conjugates with antisense morpholino oligomers. The conjugates were evaluated for cellular uptake, as determined by flow cytometry; antisense activity, as determined by a splice correction assay (Kang, Cho *et al*. 1998); and cellular toxicity, as determined by MTT cell viability, propidium iodide membrane integrity and hemolysis assays, and microscopic imaging.

The transport peptides in Table 1 include: oligoarginine sequences R₈ and R₉; sequences having RXR, RX and RB repeats (where X is a 6-aminohexanoic acid residue and B is a β-alanine residue); related sequences containing D-arginine, shown as "r", (r₈, (rX)₈, (rXR)₄, (rXr)₄ and (rB)₈); and sequences containing a combination of subsequences (RXR) and (RBR) or a combination of subsequences (RX) and (RB) ("mixed series"). As noted above, an additional B or XB residue is typically employed as a linkage to the attached molecule.

**Table 1. Exemplary Cell-Penetrating Peptides**

| Name (Designation) | Sequence | SEQ ID NO.^{a} |
|---|---|---|
| **Oligoarginines** | | |
| R₈-XB(A; 8) | RRRRRRRR-XB | 3 |
| r₈-XB | rrrrrrrr-XB | 4 |
| R₉-XB | RRRRRRRRR-XB | 5 |

| **Oligo (RX), (RXR), and (RB) series, including D-arginine** | | |
|---|---|---|
| (RX)₈-B | RXRXRXRXRXRXRXRX-B | 6 |
| (rX)₈-B | rXrXrXrXrXrXrXrX-B | 7 |
| (RX)₇-B | RXRXRXRXRXRXRX-B | 8 |
| (RX)₅-B | RXRXRXRXRX-B | 9 |
| (RX)₃-B | RXRXRX-B | 10 |
| (RXR)₄-XB (P007; 5) | RXRRXRRXRRXR-XB | 11 |
| (rXR)₄-XB | rXRrXRrXRrXR-XB | 12 |
| (rXr)₄-XB (D-P007) | rXrrXrrXrrXr-XB | 13 |
| (RB)g-B (0) | RBRBRBRBRBRBRBRB-B | 14 |
| (rB)₈-B | rBrBrBrBrBrBrBrB-B | 15 |
| (RB)₇-B | RBRBRBRBRBRBRB-B | 16 |
| (RB)₅-B | RBRBRBRBRB-B | 17 |
| (RB)₃-B | RBRBRB-B | 18 |

| **(RX), (RXR), (RB), and (RBR) mixed series** | | |
|---|---|---|
| (RXRRBR)₂-XB (B; 3b; CP06062) | RXRRBRRXRRBR-XB | 19 |
| (RXR)₃RBR-XB (C; 4c) | RXRRXRRXRRBR-XB | 26 |
| (RB)₅RXRBR-XB (D; 2) | RBRBRBRBRBRXRBR-XB | 20 |
| (RBRBRBRX)₂-X (E; 3c) | RBRBRBRXRBRBRBRX-X | 21 |
| X(RB)₃RX(RB)₃R-X (F; 3a) | XRBRBRBRXRBRBRBR-X | 22 |
| (RBRX)₄-B (G; 4b) | RBRXRBRXRBRXRBRX-B | 23 |
| (RB)₄(RX)₄-B (H; 4a) | RBRBRBRBRXRXRXRX-B | 24 |
| RX(RB)₂RX(RB)₃R-X (I; 3d) | RXRBRBRXRBRBRBRX-X | 25 |
| (RB)₇RX-B | RBRBRBRBRBRBRBRX-B | 27 |

| | | |
|---|---|---|
| ^{a}Sequences assigned to SEQ ID NOs do not include the linkage portion (X, B, or XB). | | |

### III. Cellular Uptake of Peptide-Oligomer Conjugates

Cellular uptake of peptide-PMO conjugates, where the PMO was a 3'-carboxy fluorescein-tagged PMO (PMOF), was investigated using flow cytometry. A treatment concentration of 2 µM was used because none of the conjugates caused any detectable cytotoxicity at this concentration, as demonstrated by MTT and PI uptake assays (below). After incubation with conjugate, cells were treated with trypsin (Richard, Melikov *et al*. 2003) to remove membrane-bound conjugate. To determine the effect of serum on cellular uptake of the various conjugates, uptake evaluation assays were carried out in medium containing various concentrations of serum.

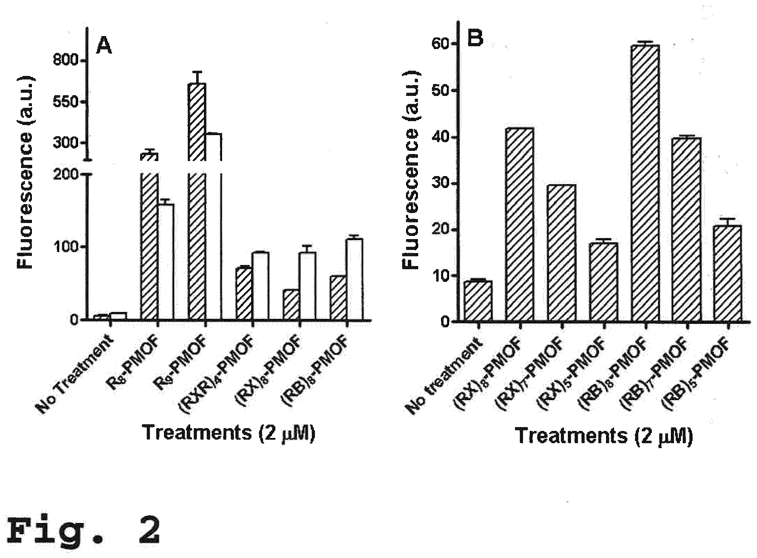

As shown in Figs. 2A-B, cellular uptake of the conjugates increased with the number of arginine residues in the transport peptide and generally decreased with X and/or B residue insertion. For example, the oligoarginine R₉-PMOF conjugate had a mean fluorescence (MF) value of 662, nearly 3-fold higher than that of Rg-PMOF. Insertion of an X or B residue in the R₈ sequence reduced uptake of the respective conjugates, as shown by MF values for conjugates of R₈ (234), (RX)₈ (42), (RXR)₄ (70), and (RB)g (60) (Fig. 2A). The number of RX or RB repeats also affected cellular uptake, with conjugates having fewer RX or RB repeats generating lower MF values (Fig. 2B).

While the addition of 10% serum to the medium caused a decrease in the uptake of the oligoarginine R₈- or R₉-PMOF conjugates, it increased uptake of conjugates containing RX, RB or RXR motifs (Figs. 2A and 2C). For example, the presence of serum reduced the MF of R₉- and R₈-PMOF from 662 and 234 to 354 and 158, respectively, but it increased the MF of (RX)₈-, (RXR)₄-, and (RB)₈-PMOF from 41, 70 and 60 to 92, 92, and 111, respectively. These differences were statically significant (Fig. 2A). However, higher serum concentrations (30% and 60%) decreased the uptake of both (RXR)₄-PMOF and oligoarginine-PMOF.

Arginine stereochemistry (D vs. L) had little effect on uptake of the peptide-PMOF conjugates. Uptake as shown by MF values of R₈-, (RB)₈- and (RX)g-PMOF conjugates was not significantly different from their respective D-isomer conjugates, r₈-, (rB)g- and (rX)₈-PMOF (data not shown).

### IV. in vitro Nuclear Antisense Activity

The effectiveness of the subject peptides in transporting an attached molecule to the nucleus of a cell was determined in a splicing correction assay (Kang, Cho *et al*. 1998), where the attached compound is a steric-blocking antisense oligomer (AO), in this case a PMO. This assay utilizes the ability of the oligomer to block a splice site created by a mutation in order to restore normal splicing. Specifically, the luciferase coding sequence is interrupted by the human β-globin thalassemic intron 2, which carries a mutated splice site at nucleotide 705. HeLa cells were stably transected with the resulting plasmid and designated pLuc705 cells. In the pLuc705 system, the oligomer must be present in the cell nucleus for splicing correction to occur. Advantages of this system include the positive readout and high signal-to-noise ratio. With this system, the relative efficiencies of various transport peptides to deliver an AO with sequence appropriate for splice-correction to cell nuclei can be easily compared.

As described below, the subject carrier peptide-PMO conjugates display higher activity in cell nuclei, and are less affected by serum and more stable in blood, than oligoarginine-PMO conjugates.

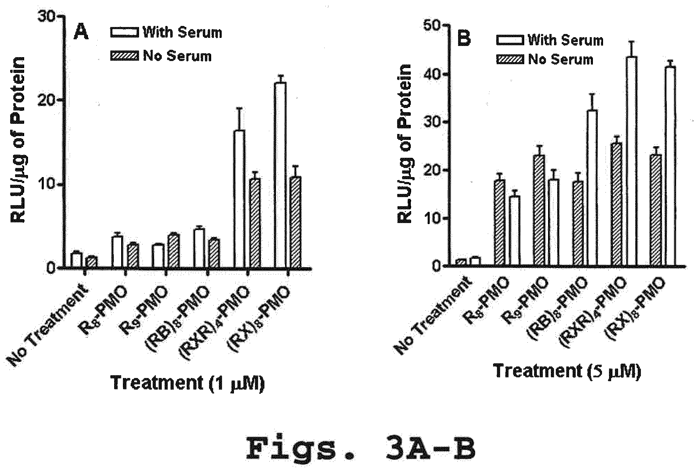

Oligoarginine, RX, RXR and RB panels (see Table 1). The peptide-PMO conjugates with the highest nuclear antisense activities in this series were found to be (RXR)₄- and (RX)₈-PMO (where, as noted above, R is arginine, and X in these peptides is 6-aminohexanoic acid). Figures 3A and 3B show luciferase activity normalized to protein of cells treated with various conjugates at 1 µM and 5 µM for 24 hr. At both concentrations, (RX)₈- and (RXR)₄-PMO were more effective than the other conjugates tested, with the difference more prominent in serum-containing medium at 1 µM than at 5 µM. Cells treated with 1µM of either conjugate exhibited luciferase activity at a level 10-15 fold over background, while the remaining conjugates yielded about a 2-4 fold increase over background (Figure 3A). At 5 µM, all conjugates generated higher luciferase activity than at 1 µM, with (RX)₈-PMO and (RXR)₄-PMO again the most effective, followed by (RB)₈-PMO (Figure 3B).

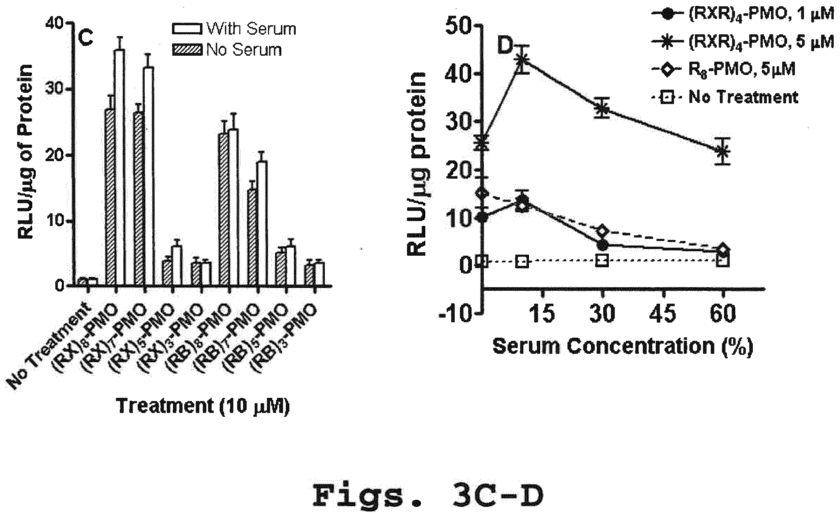

Figure 3C shows that, at 10 µM, the activity of RX or RB conjugates decreased as the number of RX or RB repeats (i.e. length) in the transport peptide decreased. The peptides with three or five RX or RB repeats generated much lower luciferase activity than those with seven or eight repeats.

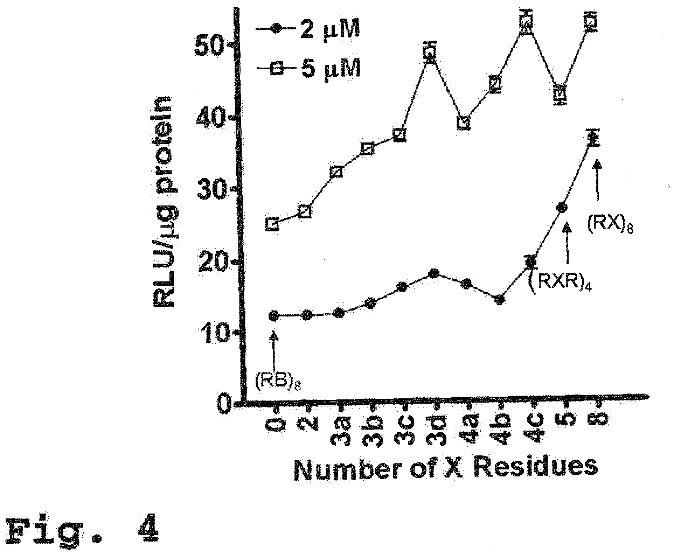

Number and position of X residues. In order to investigate the effect of the number and position of X residues on the activity of conjugates, eleven peptide-PMO conjugates, where the peptide component contained 0, 2, 3, 4, 5, or 8 X (6-aminohexanoic acid) residues, were compared (SEQ ID NOs: 14, 20, 22, 19, 21, 25, 24, 23, 26, 11 and 3 as shown in Table 1). The data (shown as lucifrase activity in the assay described above) is presented in Figure 4.

Generally, peptides containing a higher number of X residues had higher transport activities. At 2 µM, (RX)₈-PMO (eight X residues) had the highest activity, followed by (RXR)₄-PMO (five X residues), and the conjugates with fewer X residues had lower activities.

At 5 µM, three conjugates containing three (I; SEQ ID NO: 25), four (C; SEQ ID NO: 26) and eight ((RX)₈) 6-aminohexanoic acid residues had the highest activities, suggesting that the position of X residues affects activity.

Serum effect on activity. The effect of serum on the antisense activity of the conjugates was dependent on the peptide sequences, as shown in Figs. 3A-3D. Addition of 10% serum to the medium decreased the activity of oligoarginine-PMO conjugates (R₈-PMO and R₉-PMO) but increased activity of conjugates containing RXR, RX and RB repeats. The addition of 10% serum nearly doubled the luciferase activity of (RXR)₄-, (RX)₈- and (RB)₈-PMO at 5 µM (Figure 3B). This effect was further investigated for (RXR)₄-PMO up to 60% serum (see Fig. 3D). While the activity almost doubled as the serum concentration increased from 0% to 10%, it gradually decreased as the serum concentration increased to 60%, at which activity was similar to that in 0% serum (which was still significantly above background). This "up and down" profile was also observed with the 1 µM (RXR)₄-PMO treatment. Unlike (RXR)₄-PMO, the luciferase activity of R₈-PMO or R₉-PMO consistently decreased as the serum concentration increased, with an approximately 30% reduction in 10% serum and no activity in 60% serum (Fig. 3D). R₈-PMO or R₉-PMO did not display any detectable activity at 1 µM, regardless of the serum concentration (Fig. 3A).

### V. in vivo Nuclear Antisense Activity

Various transport peptides were conjugated to PMO, and the resulting conjugates (P-PMOs) were tested for their ability to transport the PMO into various tissues, in accordance with the invention, as described further in Materials and Methods, below. Briefly, conjugates were administered for four consecutive days. The *in vivo* uptake of the P-PMOs was determined by targeting the PMO (SEQ ID NO: 1) to an aberrantly spliced mutated intron in the EGFP-654 gene in an EGFP-654 transgenic mouse model (Sazani, Gemignani *et al*. 2002). In this model, cellular uptake of the EGFP-654 targeted P-PMOs can be evaluated by RT-PCR detection of restored EGFP-654 mRNA splice product and functionally restored EGFP in tissues harvested after IP administration of P-PMO.

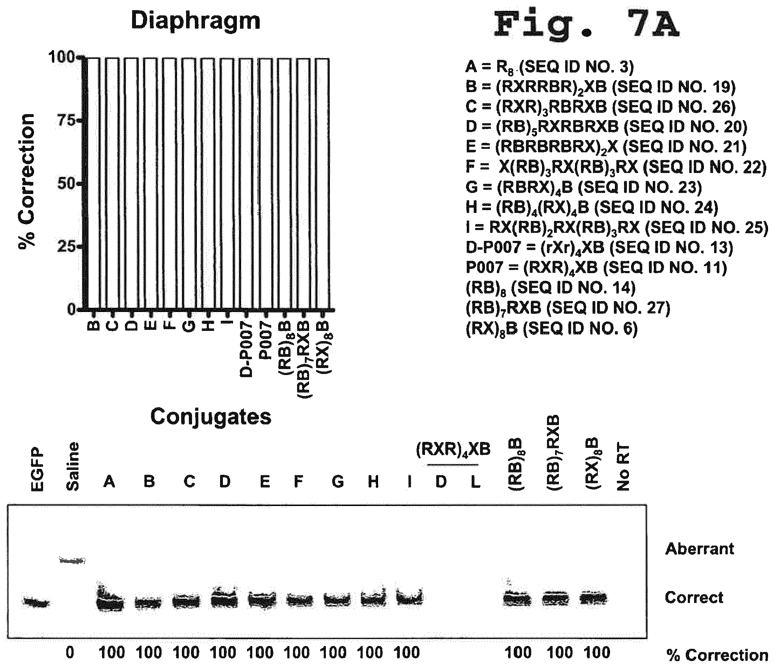

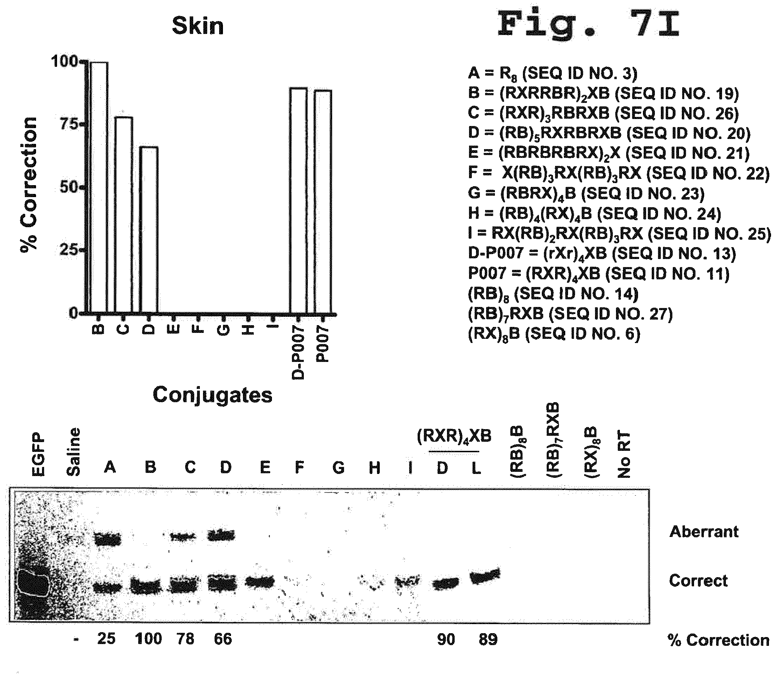

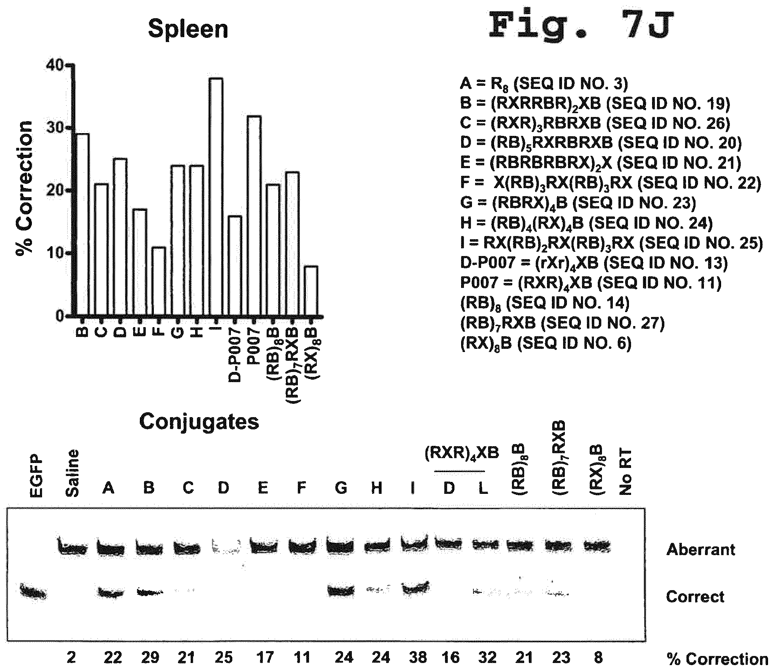

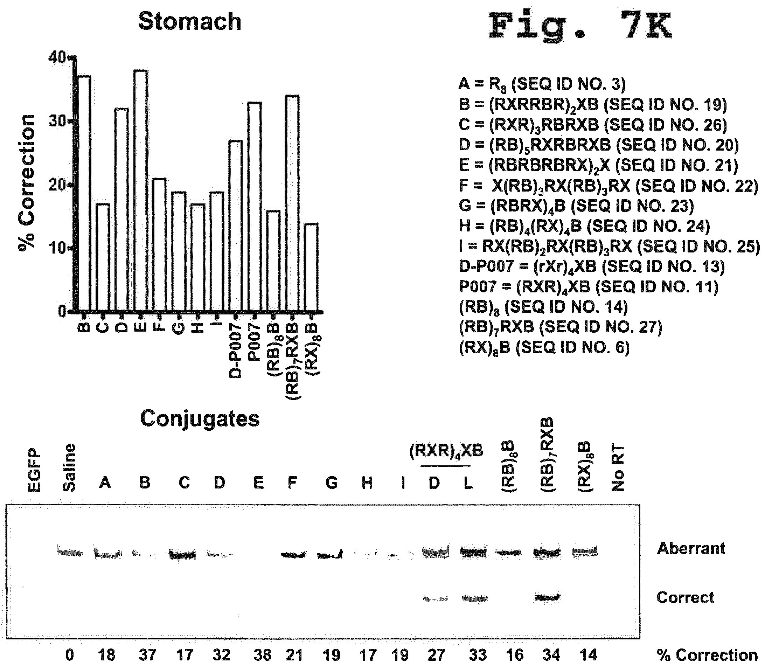

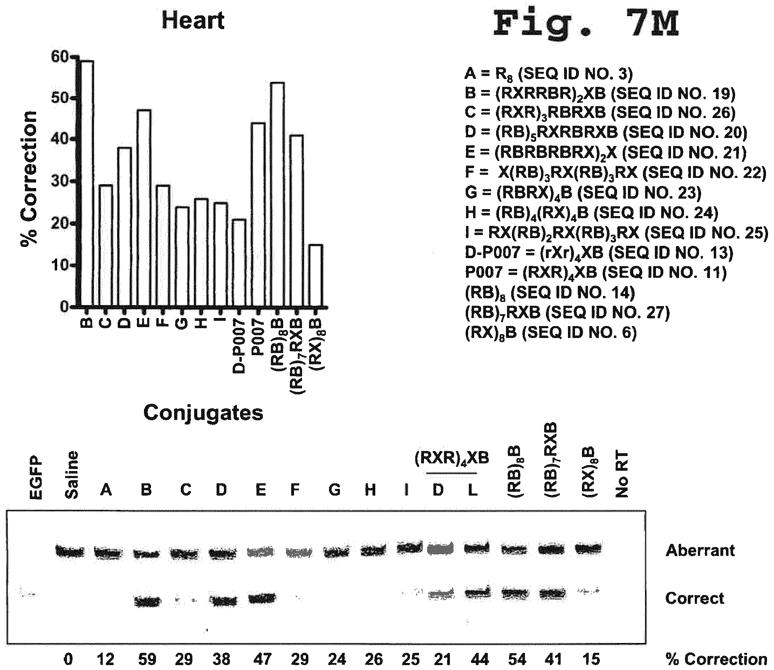

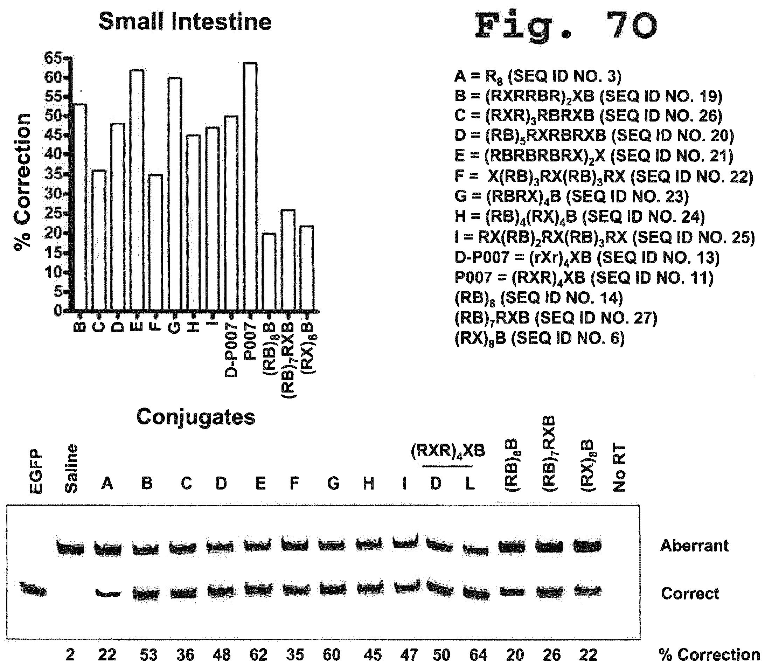

As shown in Figures 7A-P, P-PMOs containing various transport peptides displayed selective uptake by specific tissues. For example, a conjugate containing the transport peptide (RXRRBR)₂-XB (SEQ ID NO: 19) displayed selective uptake into heart, muscle, lungs, small intestine, colon, stomach, skin, and bone marrow, while uptake into other organs was greatly reduced in comparison. A conjugate containing the peptide (RBRBRBRX)₂-X (SEQ ID NO: 21) displayed selective uptake into heart, muscle, liver, small intestine, stomach, and mammary gland, while uptake into other organs was greatly reduced in comparison. A further P-PMO having the transport peptide (RB)₄(RX)₄-B (SEQ ID NO: 24) displayed selective uptake into colon, bone marrow, and brain, while uptake into other organs was greatly reduced in comparison. Optimal tissue uptake (indicated by a *) for various transport peptides is summarized in Table 2 below.

As shown herein, the invention provides carrier peptide-PMO conjugates that are superior to oligoarginine-PMO conjugates in the following aspects: they display higher activity in cell nuclei, are less affected by serum and are more stable in blood. The toxicity of the X/B containing carrier peptides can be reduced by keeping the number of X residues between 3-4 while still maintaining a reasonable delivery efficacy and stability. Further manipulation of the X/B content and ordering relative to the arginine residues can provide tissue specific delivery.

**Table 2: Carrier Peptide Uptake in Tissue**

| Tissue (Optimal % Correction) | Optimal Tissue Targeting Peptides: SEQ ID NO: (see Table 1) | | | | | | | | | | | | | |
|---|---|---|---|---|---|---|---|---|---|---|---|---|---|---|
| | 19 | 20 | 21 | 22 | 23 | 24 | 25 | 11 | 13 | 14 | 27 | 6 | 3 | 26 |
| Heart (>50%) | * | | | | | | | | | * | | | | |
| Leg muscles (≥75%) | * | * | * | | * | | * | * | * | | | * | | |
| Liver (>50%) | * | | | | * | * | * | | * | | | | * | |
| Kidney (>50%) | | | * | | | | | * | * | * | * | | | |
| Lungs (>30%) | * | | | | | | | * | | * | | | | |
| Sm. Int. (>50%) | * | | * | | * | | | * | | | | | | |
| Colon (>50%) | * | * | | | | * | * | | | | * | | | |
| Stomach (>30%) | * | * | * | | | | | * | | | * | | | |
| Mammary Gland (>75%) | | | | | | | | | | * | * | * | | |
| Thymus (>50%) | | | | | | | | * | | | | | | |
| Spleen (>30%) | | | | | | | * | * | | | | | | |
| Ovary (>50%) | | | | | * | | | | | | | | | |
| Skin (>75%) | * | | | | | | | * | * | | | | | * |
| Bone Marrow (>20%) | | | | | | | | | | | * | | | |
| Brain (>2%) | | | | | | * | | | * | | | | | |

### VI. Cellular Toxicity of Carrier Peptide-PMO Conjugates

The cellular toxicity of the various peptide-PMO conjugates was determined by MTT-survival, propidium iodine (PI) exclusion, hemolysis assays, and microscopic imaging. The MTT and PI exclusion assays measure metabolic activity and membrane integrity of cells, respectively. The hemolysis assay determines compatibility with blood. Microscopic images were used to verify the MTT results and observe the general health of the cells. As detailed below, the conjugates generally showed low toxicity, with those containing (RX)₈ and (RXR)₄ having the highest levels of toxicity.

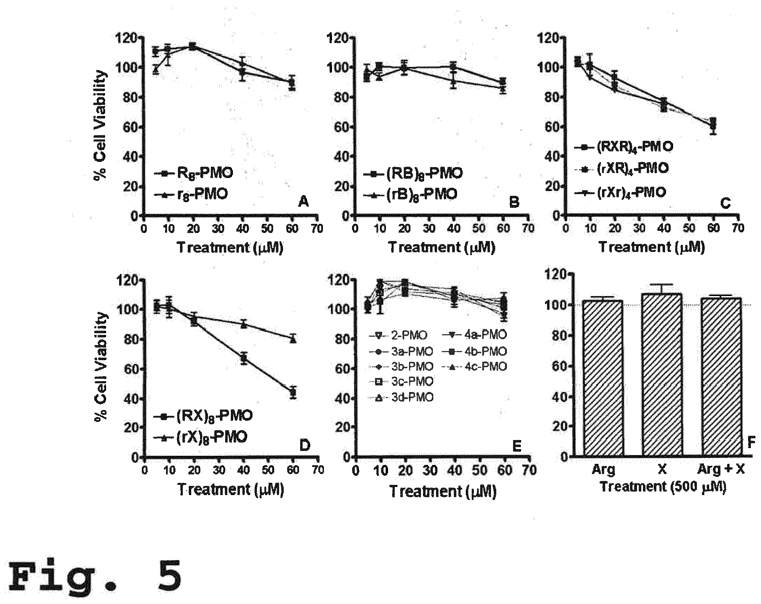

*MTT assay* (Figs. 5A-F). pLuc705 cells were treated at concentrations ranging from 2-60 µM for 24 hr. As shown in Figure 4, all conjugates, with the exception of those containing (RX)₈ and (RXR)₄, had no toxicity at up to 60 µM. The (RX)₈ and (RXR)₄ conjugates exhibited no toxicity up to 10 µM, while at higher concentrations they reduced cell viability in a concentration-dependent manner, with (RX)₈ being more toxic than (RXR)₄ (Figs. 5C-D).

Replacement of L-arginine with D-arginine in R₈-, (RB)₈- and (RXR)₄-PMO did not change the viability profiles of these conjugates (Figs. 5A-C). Surprisingly, the L→Dreplacement in (RX)₈-PMO decreased the toxicity (Fig. 5D).

The eight conjugates containing peptides with fewer than five X residues did not inhibit cell proliferation up to 60 µM (Fig. 5E). Monomers of R or X, individually or in combination, at 500 µM each, produced no inhibition of cell proliferation (Fig. 5F).

The toxicities of the conjugates (RXR)₄-PMO, RX(RB)₂RX(RB)₃RX-PMO (peptide SEQ ID NO: 25) and (RXR)₃RBR-PMO (peptide SEQ ID NO: 26) were also evaluated in a human liver HepG2 cells. Of these, only (RXR)₄-PMO caused dose-dependent inhibition of cell proliferation, while the other two conjugates had no toxicity up to 60 µM, the highest concentration tested in this study.

*Microscopic images.* Images of cells treated with 60 µM of the conjugates correlated well with the MTT cell viability data. Cells treated with (RX)₈-PMO and (RXR)₄-PMO appeared rounded and detached from the culture well, and appeared to have fewer live cells. Interestingly, cells treated with (rX)₈-PMO appeared to have normal morphology and cell density. The replacement of one X of (RXR)₄-PMO with one B reduced toxicity significantly; i.e., cells treated with (RXR)₃RBR-PMO (peptide SEQ ID NO: 26) had similar density and morphology to the vehicle-treated cells.

*Propidium iodine exclusion assay.* The effect of the conjugates on integrity of cell membranes was investigated by a propidium iodine (PI) exclusion assay. PI can permeate only unhealthy/damaged membranes; therefore, positive PI fluorescence indicates compromised cell membranes. Only (RXR)₄- PMO and (RX)₈-PMO conjugates were found to significantly affect membrane integrity at higher concentrations (up to 60 µM tested).

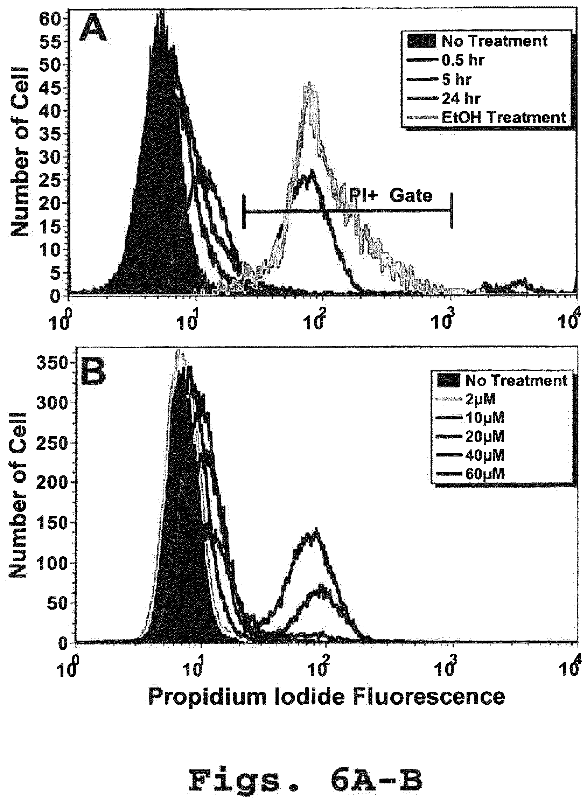

Figure 6A shows the histograms of pLuc705 cells treated with (RXR)₄-PMO at 60 µM for 0.5, 5 and 24 hr. The PI positive (PI+) region was defined by the cells permeabilized with ethanol (positive control) as indicated by the gate in the histogram. The PI histogram shifts from the PI-negative region to PI-positive region in the longer incubations, indicating the conjugate caused membrane leakage in a time-dependent manner. The 0.5 hr- and 5 hr-treatments caused a slight shift towards the PI+ region, while the 24 hr-treatment produced a distinct peak which corresponds to 57% of cells that were in the PI+ region.

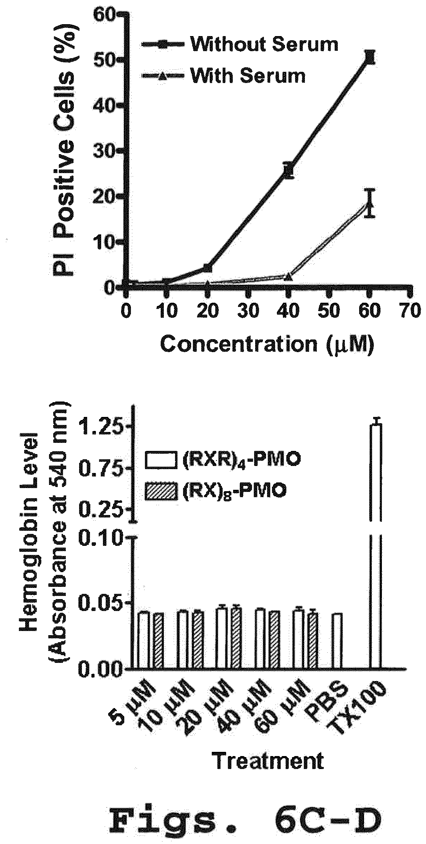

Figure 6B shows the histograms of cells treated with (RXR)₄-PMO at concentrations of 2, 10, 20, 40 and 60 µM for 24 hr. There was no significant PI uptake at concentrations up to 20 µM. At higher concentrations, the PI+ population appeared, and the percentage of PI+ cells increased as the treatment concentration increased, indicating that there were more leaking cells at the higher treatment concentration. Similar concentration- and time-dependent PI uptake profiles were observed for (RX)₈-PMO, but not for (RB)₈-PMO and the remaining conjugates. Addition of 10% serum to the treatment medium significantly reduced membrane toxicity for the (RXR)₄- (Figure 6C) and (RX)₈-PMO conjugates.

*Hemolysis assay.* The (RXR)₄- and (RX)₈-PMO conjugates were tested in a hemolysis assay and found to be compatible with red blood cells. Fresh rat red blood cells were treated with the conjugates at 60 µM, PBS (background) or 0.005% TX-100 (positive control). The supernatants of conjugate- and PBS-treated samples had small and similar amounts of free hemoglobin released, far lower than that of the TX-100-treated samples (Figure 6D).

### VII. Screening Methods

As shown herein, transport peptides consisting of varying sequence motifs containing arginine, including D-arginine, β-alanine, and 6-aminohexanoic acid and homologs are often tissue selective, in that different sequence peptides provide superior transport in different selected tissues.

Accordingly, libraries of different-sequence peptides can be used in a method for identifying a cell-penetrating peptide useful for targeting a therapeutic compound to a selected mammalian tissue. The method comprises the steps of:
(a) forming a library of peptide conjugates composed of
   (i) a plurality of different-sequence peptides, each 8 to 25 amino acid residues in length and consisting of subsequences selected from the group consisting of RXR, RX, RB, and RBR; where R is arginine, B is β-alanine, and each X is independently -C(O)-(CHR¹)ₙ-NH-, where n is 4-6 and each R¹ is independently H or methyl, such that at most two R¹'s are methyl, and
   (ii) covalently coupled to each peptide, via an X, B, or XB linkage, a marker compound whose concentration can be assayed in the cells of the selected tissue;
(b) administering each peptide conjugate to a mammalian subject;
(c) assaying the level of the marker compound in cells of the selected tissue, after a period sufficient for localization of the administered peptide conjugate in the selected tissue of the mammalian subject; and
(d) selecting a cell-penetrating peptide useful for targeting a therapeutic compound to the selected mammalian tissue, based on its ability to produce highest or near-highest levels of marker compound, relative to other peptides in the plurality, in the selected tissue.

In some cases, a peptide having high transport activity in a broad variety of tissues is sought. Alternatively, a peptide having high transport activity in a particular tissue relative to other tissues may be sought. Thus, the assaying of step (c) above may comprise assaying the level of the marker compound in cells of a plurality of selected tissues, and the selecting of step (d) may comprise selecting a cell-penetrating peptide useful for targeting a therapeutic compound to a selected tissue of the plurality, based on its ability to produce highest or near-highest levels of marker compound in the selected tissue, relative to other peptides in the plurality of peptides, and/or relative to other tissues in the plurality of tissues.

In the different sequence peptides, each X is preferably a 5-aminopentanoic acid residue or, more, preferably, a 6-aminohexanoic acid residue, and each peptide preferably contains at least three such X residues. Preferred classes of peptides include those whose sequence is made up of a combination of subsequences (RXR) and (RBR), or a combination of subsequences (RX) and (RB). In each case, the peptide preferably includes at least three, and more preferably at least four, X residues.

The library may include, for example, peptides selected from the group having sequences identified by SEQ ID NOs: 6-27, preferably SEQ ID NOs: 19-27.

Typically, one or two N-terminal amino acid residues selected from the group consisting of 6-aminohexanoic acid, 5-aminopentanoic acid, and β-alanine are employed as a linkage from the peptide to the marker compound.

Preferably, the marker compound used for screening is structurally similar to the therapeutic molecule(s) desired to be transported into cells. In a preferred embodiment, the marker compound and the molecules to be transported are oligomeric antisense compounds, particularly morpholino antisense compounds. A useful oligomeric marker compound to be conjugated to the peptides is a fluorescently labeled or unlabeled oligonucleotide analog, e.g. a PMO as described herein. Cells of the selected tissues can be examined by well known methods to assay for the presence of internalized fluorescent marker and hence determine the extent of internalization.

An mRNA splice correction assay having a visual readout, such as that described in Materials and Methods below, can also be used to assay for nuclear internalization. Alternatively, the oligomer marker compound may be an antisense oligonucleotide effective to produce exon skipping in a selected cellular protein, where the assaying step includes examining the protein products produced by cells of the selected tissue for the presence of the selected cellular protein in a truncated form indicating such exon skipping. An exemplary screening using this method is also described in Materials and Methods below.

### VIII. Exemplary Therapeutic Applications

Peptides identified as having desirable tissue delivery characteristics, e.g. by the screening methods described above, can be used for preparing a therapeutic conjugate for use in treating a disease condition associated with a selected tissue in a mammalian subject. Accordingly, a transport peptide for a selected tissue, selected by the above described screening methods, is identified, along with a therapeutic compound which is effective against the disease condition when localized in cells of the selected tissue. The therapeutic compound can then be conjugated to a terminus, preferably the N-terminus, of the selected transport peptide.

The transport peptides described herein find particular use in applications involving difficultly soluble or otherwise poorly transported therapeutics. In addition to the use of the carrier peptides for delivery of antisense oligonucleotides and their analogs, the compounds of the present invention can be used to target therapeutically useful molecules that otherwise are limited in their ability to enter the cells of target tissues, such as, for example, paclitaxel (Taxol®) and doxorubicin.

Phosphorodiamidate-linked morpholino oligomers (PMO) have been shown to be taken up into cells and to be more consistently effective *in vivo*, with fewer nonspecific effects, than other widely used oligonucleotide chemistries (see e.g. P. Iversen, "Phosphoramidate Morpholino Oligomers", in Antisense Drug Technology, S.T. Crooke, ed., Marcel Dekker, Inc., New York, 2001). However, further enhancement in uptake and targeted biodistribution is desirable, and can be achieved, as demonstrated herein, by use of the disclosed cell penetrating peptides.

The carrier peptides and conjugates of the present invention are particularly useful for targeting and delivering an antisense oligomer, such as a PMO, across both the cell and nuclear membranes to the nucleus of specific cell types, by exposing the cell to a conjugate comprising the oligomer covalently linked to a carrier peptide as described above. Such delivery allows for targeting of splice sites, which can be implemented for generating proteins with altered function. The translation start site (i.e. the AUG start codon) is another useful target for antisense therapy, as are sequences required for viral replication.

Peptide-antisense conjugates provided by the present invention find use in any indication in which delivery to specific cell types is desirable. Exemplary indications include, but are not limited to, antisense oligomers that target: P450 enzymes, for alteration of drug metabolism in the liver; *c-myc*, for treatment of polycystic kidney disease in the kidney, or to prevent coronary artery restenosis in vascular endothelium; dystrophin, for the treatment of Duchenne muscular dystrophy in cardiac and skeletal muscle tissues; myostatin, for the treatment of muscle atrophy in skeletal muscles; viruses that cause chronic infections of the liver, such as hepatitis C virus and hepatitis B virus; viruses that infect lung tissues, such as influenza and respiratory syncytial virus (RSV); TNF receptor, for the generation of a soluble isoform of the TNF receptor in the liver to inhibit TNF-α induced inflammatory arthritis; the TGF-beta gene in bone marrow, for the generation of elevated long-term repopulating hematopoietic stem cells; and intracellular parasites, such as plasmodium falciparum infection of the liver. Specific combinations of tissue specific carrier peptides and antisense oligomers to treat the above and other indications are described below.

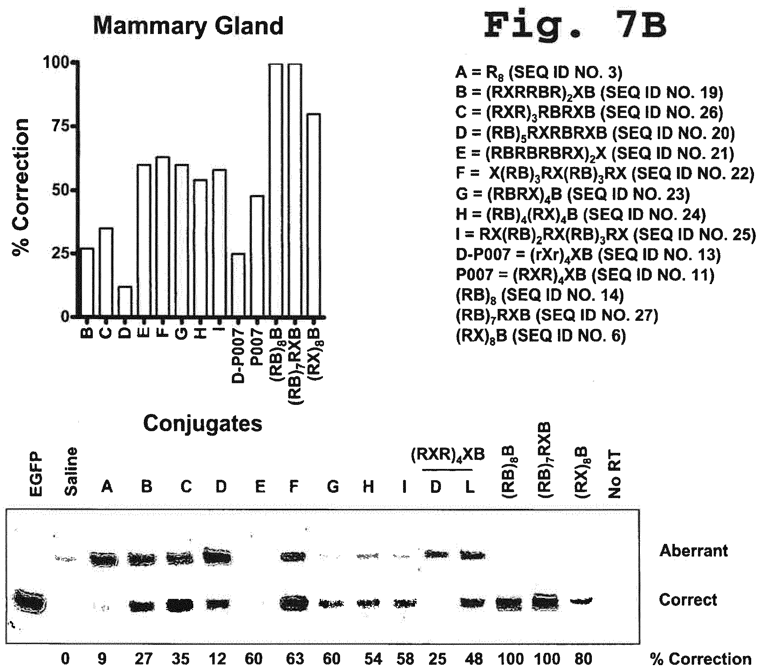

In one embodiment, a therapeutic conjugate is provided for use in treating breast cancer in a mammalian subject. As shown by the data in Fig. 7B, exemplary peptides shown to enhance transport into breast tissue include those having the SEQ ID NOs: 6, 14, 21-23, 25, and 27, and preferably SEQ ID NOs: 6, 14, 22 and 27, and the therapeutic compound may be selected from the group consisting of methotrexate, cyclophosphamide, doxorubicin, 5-fluorouracil, epirubicin, and trastuzumab (Herceptin®).

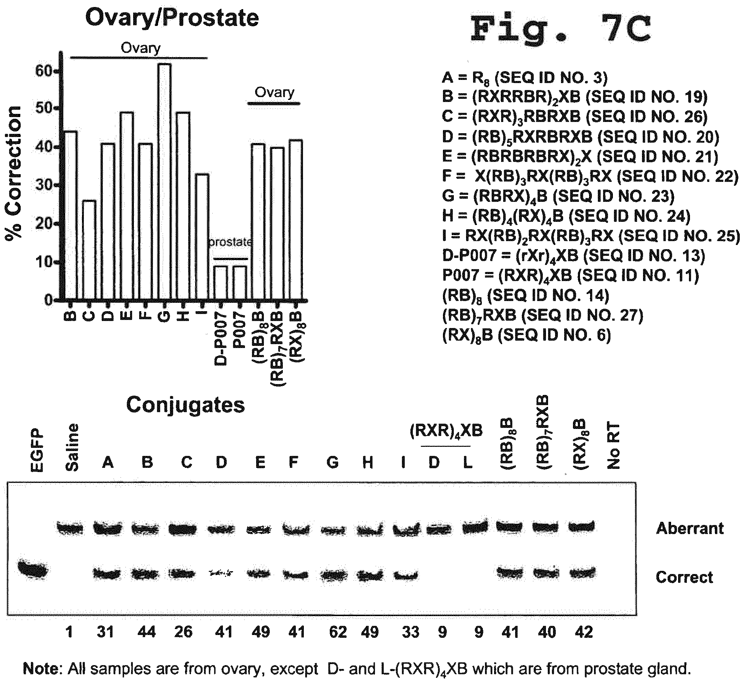

In another embodiment, a therapeutic conjugate is provided for use in treating ovarian cancer in a mammalian subject. As shown by the data in Fig. 7C, exemplary peptides shown to enhance transport into ovarian tissue include those having the SEQ ID NOs: 6, 14, 19, 21, 23, 24, and 27, and preferably SEQ ID NOs: 23 and 27, and the therapeutic compound may be selected from the group consisting of paclitaxel (Taxol®), topotecan, doxorubicin, trastuzumab (Herceptin®) and pertuzamab.

In another embodiment, a therapeutic conjugate is provided for use in treating prostate cancer in a mammalian subject. An exemplary peptide for enhancing transport into prostate tissue has the sequence identified by SEQ ID NO: 23, and the therapeutic compound may be selected from: (i) an antibody specific against prostate stem cell antigen, and (ii) an antisense oligomer targeted against human androgen receptor protein, such as a PMO having SEQ ID NO: 43 (see Sequence Table, below).

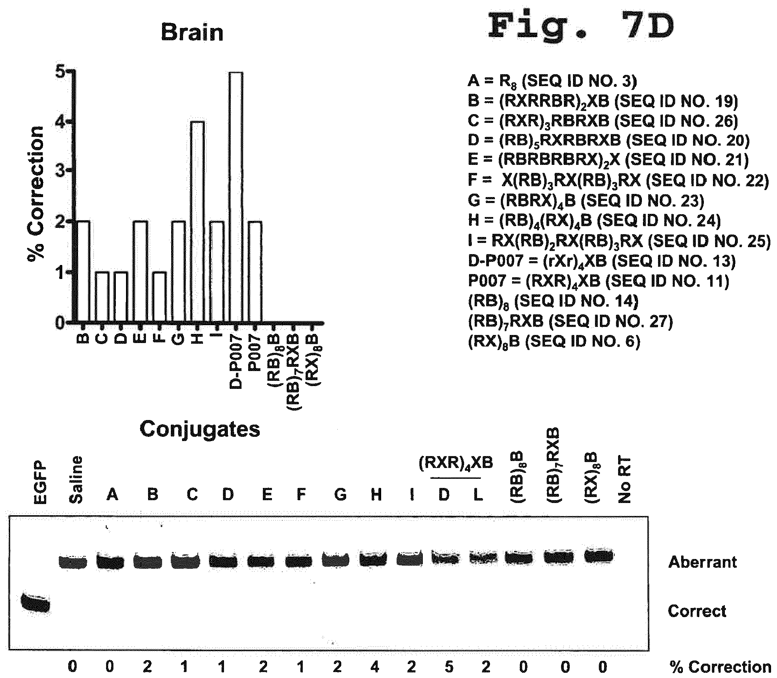

In one embodiment, a therapeutic conjugate is provided for use in treating a disease condition associated with the CNS in a mammalian subject. As shown by the data in Fig. 7D, exemplary peptides shown to enhance transport into brain tissue include those having the SEQ ID NOs: 13 and 24, and the therapeutic compound may be selected from the group consisting of: OM99-1, OM99-2, OM00-3, KMI-429, CEP-1347, Humanin, Minocycline, and valproate, for the treatment of Alzheimer's disease; CEP-1347 and bromocriptine, for the treatment of Parkinson's disease; azidothymidine, acyclovir, and antiviral antisense compounds, for the treatment of viral infections of the CNS; and penicillin, vancomycin, gentamicin, netilmicin, ciprofloxacin, and antisense antibacterial compounds, for the treatment of bacterial disease of the CNS.

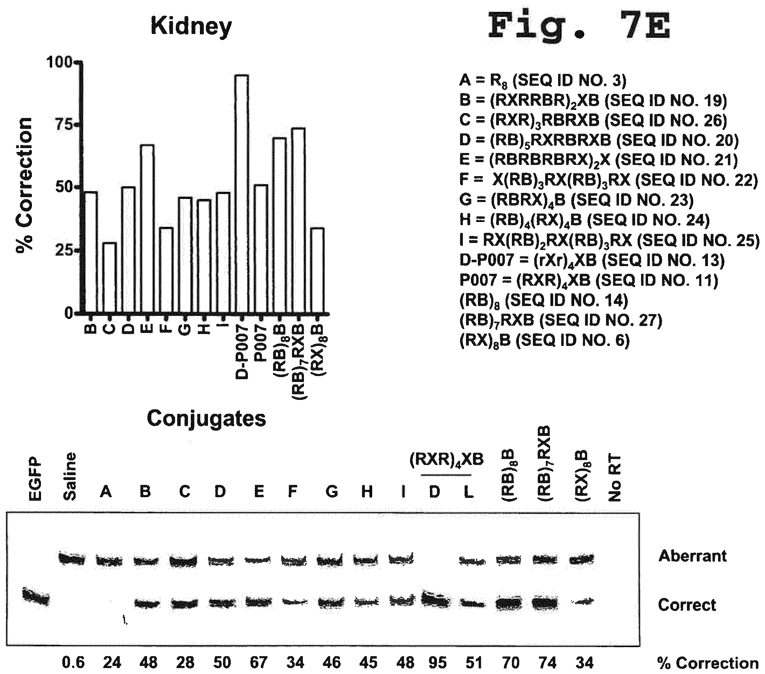

In another embodiment, a therapeutic conjugate is provided for use in treating diseases of the kidney in a mammalian subject. As shown by the data in Fig. 7E, exemplary peptides shown to enhance transport into kidney tissue include those having the SEQ ID NOs: 13, 14, 21, and 27, and preferably SEQ ID NO: 13, and the therapeutic compound may be selected from the group consisting of:
(a) gemcitabine and capecitabine, for the treatment of cancer of the kidney, and
(b) an antisense oligomer targeted against human c-myc, for the treatment of polycystic kidney disease. An exemplary oligomer is a PMO having a sequence selected from SEQ ID NOs: 31-33.

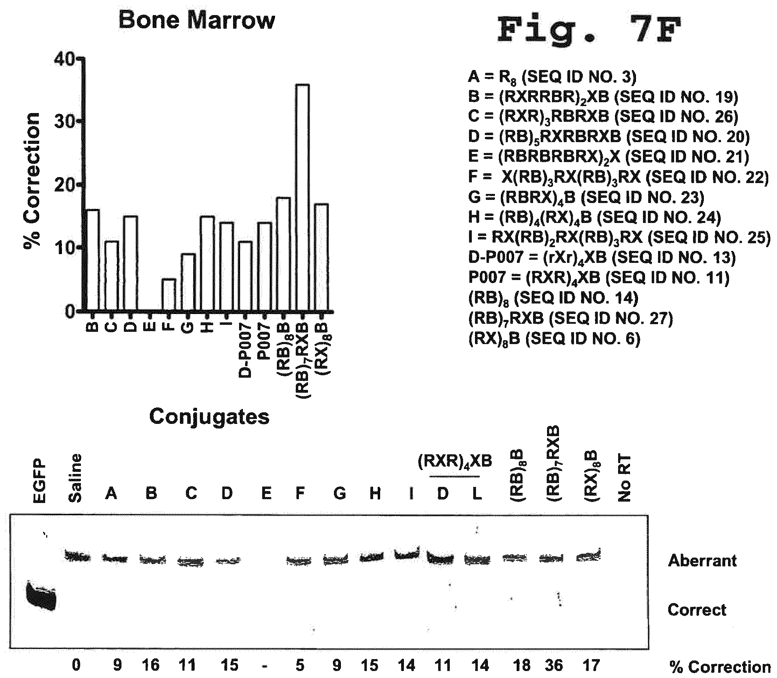

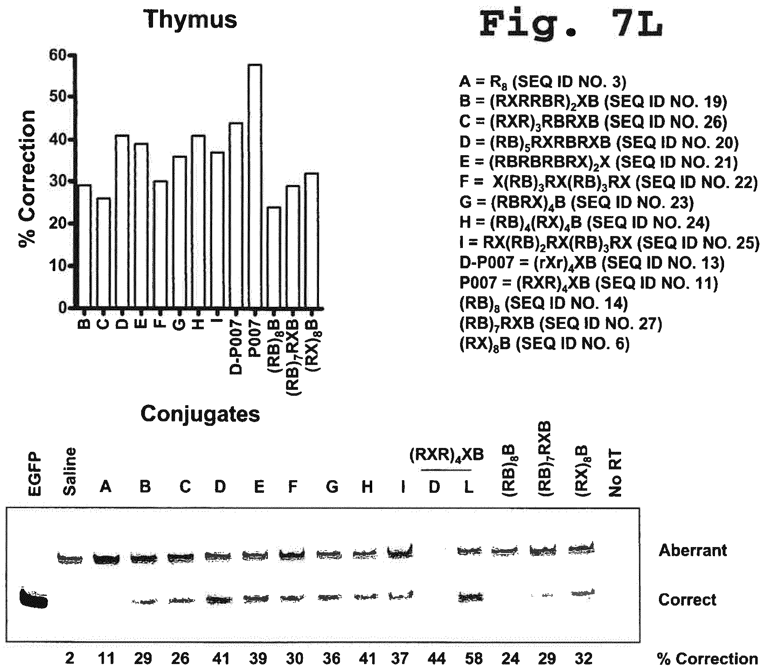

In another embodiment, a therapeutic conjugate is provided for use in enhancing stem cell proliferation and survival in peripheral blood. As shown in Fig. 7F, exemplary peptides shown to enhance transport into bone marrow include the peptides having sequences selected from SEQ ID NOs: 14, 19, and 27, and preferably SEQ ID NO: 27. As shown in Fig. 7L, exemplary peptides shown to enhance transport into thymus tissue include peptides having SEQ ID NOs: 11, 13, 20, and 24, and preferably SEQ ID NO: 11. The therapeutic compound is preferably an antisense oligomer targeted against human TGF-β, such as a PMO having a sequence selected from SEQ ID NOs: 44-46.

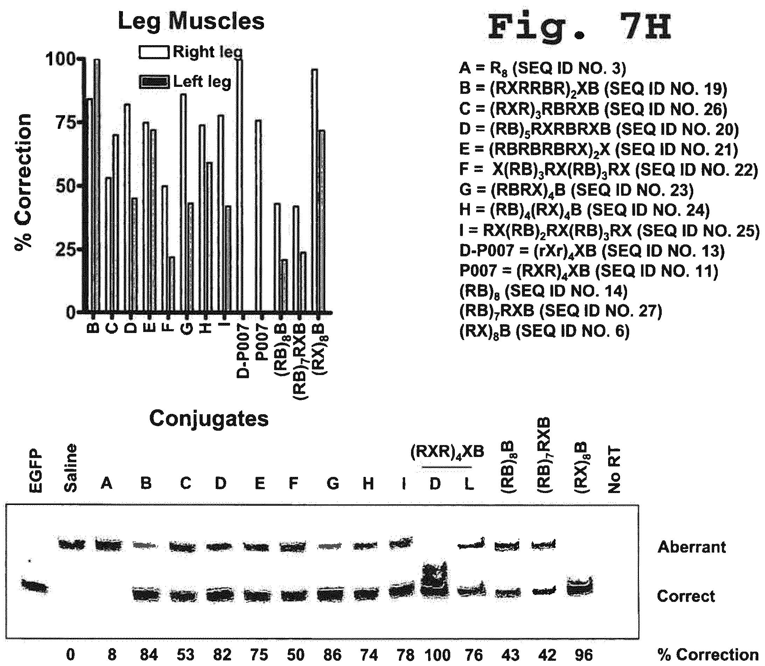

In another embodiment, a therapeutic conjugate is provided for use in treating a disease condition associated with muscle tissue in a mammalian subject. As shown by the data in Fig. 7H, exemplary peptides shown to enhance transport into muscle tissue include those having the SEQ ID NOs: 6, 13, 19, and 20, and preferably SEQ ID NOs: 6, 13, and 19, and the therapeutic compound may be selected from the group consisting of:
(a) an antisense oligomer targeted against human myostatin, such as a PMO having a sequence selected from SEQ ID NOs: 35-39, for treating a muscle wasting condition, as discussed further below; and
(b) an antisense oligomer capable of producing exon skipping in the DMD protein, such as a PMO having a sequence selected from SEQ ID NOs: 34 and 49, to restore partial activity of the protein, for treating Duchenne muscular dystrophy, which is discussed further below.

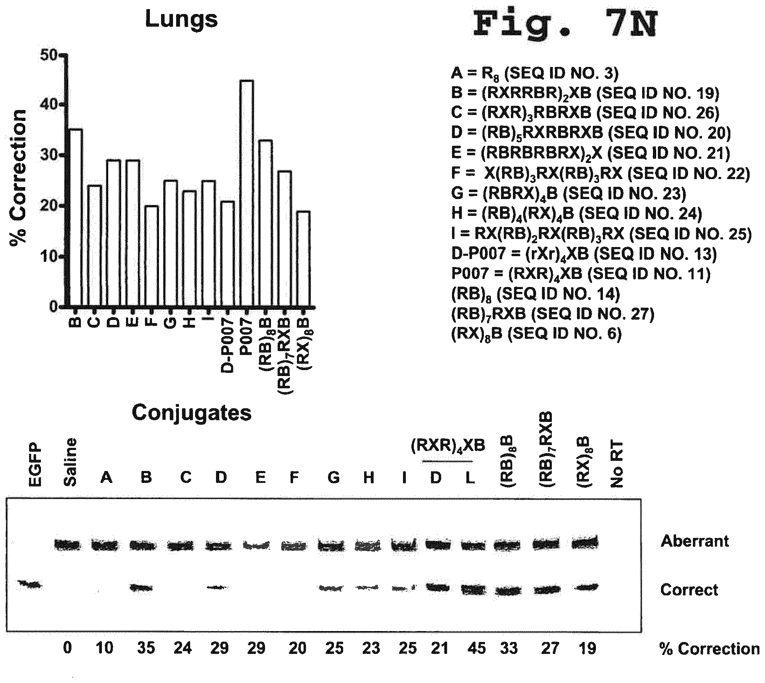

In another embodiment, a therapeutic conjugate is provided for use in treating a disease condition associated with the lungs in a mammalian subject. As shown by the data in Fig. 7N, exemplary peptides shown to enhance transport into lung tissue include those having the SEQ ID NOs: 11, 14, and 19, and preferably SEQ ID NOs: 11 and 19, and the therapeutic compound may be selected from the group consisting of:
(a) cisplatin, carboplatin, and docetaxel, for the treatment of lung cancer;
(b) an antisense oligomer targeted against bacterial 16S rRNA, such as a PMO having SEQ ID NO: 47, for treatment of bacterial respiratory infections; and
(c) an antisense oligomer targeted against influenza A virus, such as a PMO having a sequence selected from SEQ ID NOs: 41 and 42, or against respiratory syncytial virus, such as a PMO having SEQ ID NO: 48, for treatment of bacterial respiratory infections, which is discussed further below.

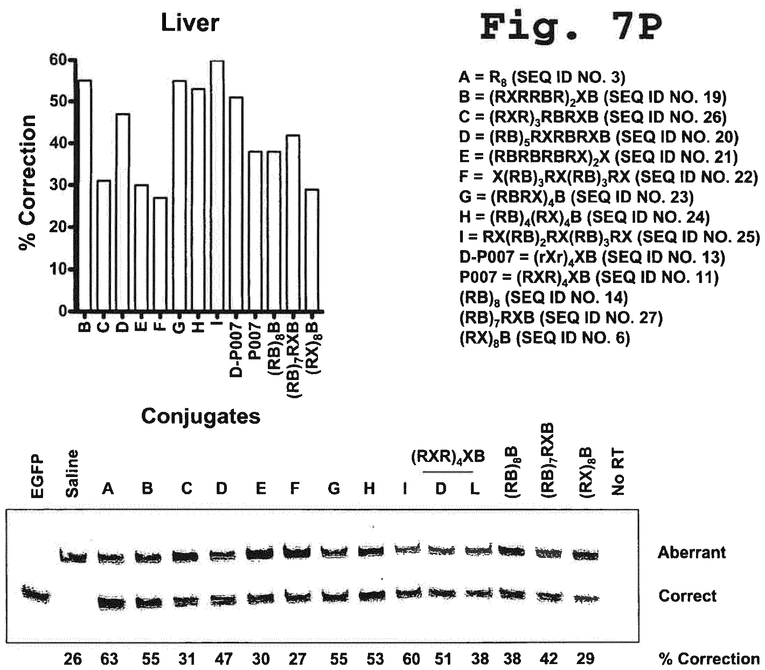

In another embodiment, a therapeutic conjugate is provided for use in treating a disease condition associated with liver tissue in a mammalian subject. As shown by the data in Fig. 7P, exemplary peptides shown to enhance transport into liver tissue include those having the SEQ ID NOs: 13, 19, 20, and 23-25, and preferably SEQ ID NOs: 19 and 23-25, and the therapeutic compound may be selected from the group consisting of:
(a) doxorubicin, 5-fluorouracil, and methotrexate, for the treatment of liver cancer;
(b) an antisense oligomer targeted against a P450 enzyme, such as a PMO having a sequence selected from SEQ ID NOs: 28-30, for suppressing drug metabolism in the liver, which is discussed further below;
(c) an antisense oligomer targeted against HCV, such as a PMO having a sequence selected from SEQ ID NOs: 40 and 50, for treatment of viral hepatitis, which is discussed further below.

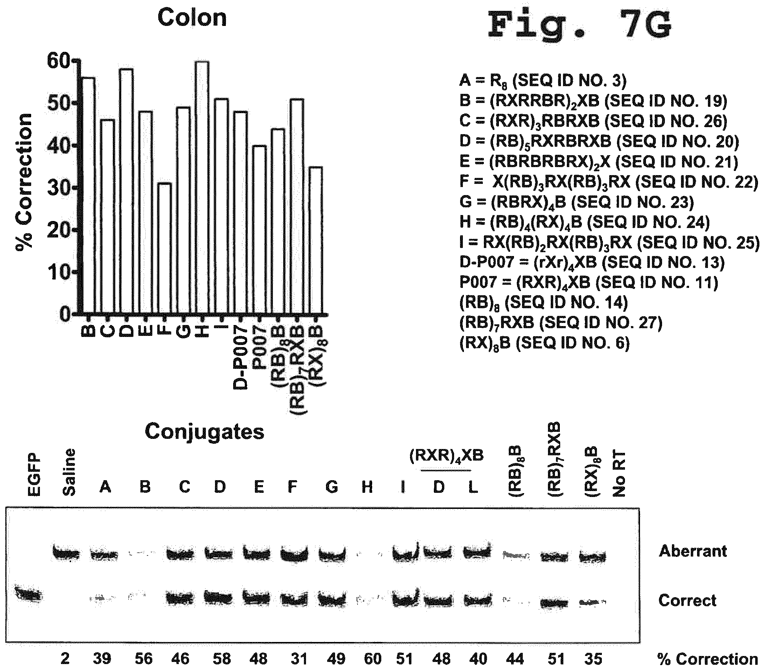

In another embodiment, a therapeutic conjugate is provided for use in treating colon cancer in a mammalian subject. As shown by the data in Fig. 7G, exemplary peptides shown to enhance transport into colon tissue include those having the SEQ ID NOs: 19, 20, 24, and 27, and preferably SEQ ID NOs: 20 and 24, and the therapeutic compound may be selected from the group consisting of 5-fluorouracil, irinotecan, oxaliplatin, bevacizumab (Avastin®), and cetuxima. In one embodiment, 5-fluorouracil can be used in combination with one of the other drugs named.

In summary, the present invention provides carrier peptide-PMO conjugates that are superior to oligoarginine-PMO conjugates for the following reasons: they display higher activity in cell nuclei, are less affected by serum and are more stable in blood. The toxicity of the X/B containing carrier peptides can be reduced by keeping number of X residues between 3-4 while still maintaining a reasonable delivery efficacy and stability. Further manipulation of the X/B content and ordering relative to the arginine residues can provide enhanced tissue specific delivery.

### A. Specific Applications of Peptide-Oligomer Conjugates

### (A1) Improved pharmacokinetics of various drugs after treatment with antisense oligomers that target CYP3A4.

In one exemplary embodiment, a carrier peptide of the present invention can be used to improve the pharmacokinetics of various drugs in patients by administering an antisense oligomer coupled to one or more of the carrier peptides described herein and targeted to CYP3A4, a gene encoding a drug-metabolizing enzyme which reduces the half-life of the drug. The antisense oligomer is effective to reduce the production of the CYP3A4 enzyme in the subject, extending the drug's half-life and effectiveness and decreasing the drugs toxicity. (See *e.g.* PCT Pubn. No. WO/2001/087286 or U.S. Appn. Pubn. No. 20040229829.) Exemplary compositions comprise CYP3A4 antisense oligomers coupled to a carrier peptide with liver specific delivery properties, as described in the current invention, that target the AUG start codon region in the mRNA or splice sites in the preprocessed RNA of the CYP3A4 gene. Exemplary carrier peptides are the B (CP06062), G, H and I peptides (SEQ ID NOs: 19 and 23-25) and preferred antisense oligomers have a sequence presented as the group consisting of SEQ ID NOs: 28-30.

*(A2) Antisense compounds for treating restenosis.* The compounds and methods of the present invention are useful in treatment of vascular proliferative disorders, such as restenosis resulting from vascular trauma. Areas of vessel injury include, for example, the vascular lumen following vascular intervention, such as coronary artery balloon angioplasty, with or without stent insertion. Restenosis is believed to occur in about 30% to 60% of lesions treated by angioplasty and about 20% of lesions treated with stents within 3 to 6 months following the procedure. (See, *e.g*., Devi, N.B. et al., Cathet Cardiovasc Diagn 45(3):337-45, 1998). Stenosis can also occur after a coronary artery bypass operation, typically in the transplanted blood vessel segments, and particularly at the junction of replaced vessels. Stenosis can also occur at anastomotic junctions created for dialysis.

A tissue-specific transport peptide conjugated to an antisense oligomer directed against *c-myc* can be used to reduce the risk of restenosis in transluminal angioplasty, such as percutaneous transluminal coronary angioplasty (PTCA) (see *e.g.* PCT Pubn. No. WO 2000/044897). Compared to oligomers not conjugated to a transport peptide, the conjugate oligomers exhibit improved delivery to vascular endothelium are expected to provide greater efficacy at lower doses in the treatment of restenosis.

Thus, the method includes administering to the patient, by local administration directly to the vessel site of injury, or by systemic delivery via intravascular administration, an *anti-c-myc* oligomer as described herein, including a targeting base sequence that is complementary to a target sequence of at least 12 contiguous bases within the AUG start site region of human *c-myc* mRNA, conjugated to a transport peptide with enhanced deliver to vascular tissue, in an amount effective to reduce the risk of restenosis in the patient. Exemplary transport peptides include those having SEQ ID NO: 21 or, preferably, SEQ ID NO: 19.

The conjugate is administered by one of:
(a) contacting the region of the vessel with a reservoir containing the antisense compound, and introducing the compound from the reservoir into the vessel by iontophoresis or electroporation;
(b) injecting the compound from the catheter directly into the region of the vessel, under pressure, through injectors contained on the surface of the catheter balloon, where said injectors are capable of penetrating the tunica media in the vessel;
(c) injecting into or contacting the region of the vessel, microparticles containing the antisense compound in entrapped form;
(d) contacting the region of the vessel with a hydrogel coating contained on the surface of the catheter balloon, and containing the antisense compound is diffusible form; and
(e) contacting the region of the vessel with a stent having an outer surface layer containing the antisense compound in diffusible form;
(f) injecting the compound by intravascular administration resulting in systemic delivery to the vascular tissues.

The antisense compound preferably has a targeting sequence having at least 90% homology to a sequence selected from the group identified by SEQ ID NOs: 31-32.

The amount of antisense compound administered may be between about 0.5 and 30 mg. The compound may be derivatized with a moiety that enhances the solubility of the compound in aqueous medium, and the compound is administered from a solution containing at least about 30 mg/ml of the antisense compound.

The compound is designed to hybridize to *c-myc* mRNA under physiological conditions with a Tm substantially greater than 37°C, *e.g*., at least 50°C and preferably 60-80°C. The compound preferably contains an internal 3-base triplet complementary to the AUG site, and bases complementary to one or more bases 5' and 3' to the start site. One preferred compound sequence is the 20-mer identified as SEQ ID NO: 31, where the CAT triplet in the sequence binds to the AUG start site, the 6 bases 3' to the CAT sequence extend in the upstream (5') direction on the target, and the 11 bases 5' to the CAT sequence extend downstream on the target.

The oligomer is employed, for example, in a coated stent, or by an *ex vivo* soaking solution for treatment of saphenous veins, or otherwise delivered to the site of vascular injury. The oligomer can also be employed by administering via systemic delivery to the site of vascular injury by intravascular injection.

In another embodiment, the antisense compound forms part of a particle composition for use in restenosis treatment. One such particle is a biodegradable particle, *e.g*., a polylactate or polyglycolic particle, containing entrapped antisense compound. The particles are preferably in the 1-5 micron range, and are useful for delivery by direct particle delivery to an angioplasty vessel site, as described below, either by being impressed into the vessel walls by pressure from a balloon against the wall, or by release from a particle carrier, such as a stent.

Alternatively, the particles can be microbubbles containing the compound in entrapped form. The particles may be delivered directly to the vessel site, that is, by contacting the vessel walls with a directly with a suspension of the particles, with compound release from the particles, which may be facilitated by exposing the vessel region to ultrasonic energy. Microbubble compositions have been found particularly useful in delivery of attached molecules, such as oligonucleotides, to areas of thrombosis or vessel injury, e.g. damaged endothelium, as well as to selected organs such as the liver and kidney. See, for example, PCT Pubn. No. WO 2000/02588, U.S. Patent Nos. 6,245,247 and 7,094,765, and U.S. Appn. Pubn. No. 20030207907.

The transport peptide may also be conjugated to a non-antisense antirestenotic compound, such as rapamycin, and the conjugate delivered in a similar manner for treatment of restenosis.

*(A3) Treatment of Duchenne muscular dystrophy.* In another embodiment, an antisense oligomer conjugated to a muscle-specific carrier peptide as described herein can be used in an improved method for treating Duchenne muscular dystrophy (DMD). Mutations in the human dystrophin gene can be removed from the processed mRNA by antisense oligomers that cause exon skipping of the exon containing the mutation. The resulting processed dystrophin mRNA can encode a functional dystrophin protein. An exemplary antisense oligomer targeted to exon 51 of the human dystrophin gene (SEQ ID NO: 34) induces skipping of exon 51. Other suitable antisense oligomers include those having SEQ ID NOs: 49 (human exon 50) and 77 (murine exon 23).

This therapeutic strategy can benefit greatly from the use of muscle-specific carrier peptides as exemplified by the B (CP06062), D-P007 and (RX)₈B peptides (SEQ ID NOs: 19, 13 and 6, respectively). As described below in section B and exemplified in Example 2, additional conjugation of a muscle-specific homing peptide enhances effectiveness of the oligomer still further.

*(A4) Treatment of muscle atrophy*. In another embodiment, an antisense oligomer as described herein can be used in a method for treating loss of skeletal muscle mass in a human subject. The steps in the method entail
(a) measuring blood or tissue levels of myostatin in the subject,
(b) administering to the subject, a myostatin-expression-inhibiting amount of an oligomer as described herein, conjugated to a carrier peptide as described herein, and having a base sequence effective to hybridize to an expression-sensitive region of processed or preprocessed human myostatin RNA transcript;
(c) by this administering, forming within target muscle cells in the subject, a base-paired heteroduplex structure composed of human myostatin RNA transcript and the antisense compound and having a Tm of dissociation of at least 45°C, thereby inhibiting expression of myostatin in said cells;
(d) at a selected time following administering the antisense compound, measuring a blood or tissue level of myostatin in the subject; and
(e) repeating the administering, using the myostatin levels measured in (d) to adjust the dose or dosing schedule of the amount of antisense compound administered, if necessary, so as to reduce measured levels of myostatin over those initially measured and maintain such levels of myostatin measured in step (d) within a range determined for normal, healthy individuals.

Where the antisense oligomer is effective to hybridize to a splice site of preprocessed human myostatin transcript, it has a base sequence that is complementary to at least 12 contiguous bases of a splice site in a preprocessed human myostatin transcript, and formation of the heteroduplex in step (c) is effective to block processing of a preprocessed myostatin transcript to produce a full-length, processed myostatin transcript. Exemplary antisense sequences are those identified by SEQ ID NOs: 35-39, and muscle specific carrier peptides are exemplified by the B (CP06062), D-P007 and (RX)₈B peptides (SEQ ID NOs: 19, 13 and 6, respectively).

*(A5) Treatment of chronic viral infections of the liver.* An exemplary antisense antiviral application of the present invention is for use in a method for the inhibition of growth of the hepatitis C virus (HCV). The inhibiting compounds consist of antisense oligomers conjugated to liver-specific carrier peptide, as described herein, having a targeting base sequence that is substantially complementary to a viral target sequence which spans the AUG start site of the first open reading frame of the HCV viral genome. The targeting sequence is complementary to a sequence of at least 12 contiguous bases of the HCV AUG start-site and IRES regions. Exemplary targeting sequences include those having at least 90% homology to SEQ ID NOs. 40 and 50, respectively. In one embodiment of the method, the oligomer is administered to a mammalian subject chronically infected with the HCV virus. See, e.g., PCT Pubn. No. WO/2005/007805 and US Appn. Pubn. No. 2003224353. Exemplary liver-specific carrier peptide for conjugating to these antisense oligomers include those represented by SEQ ID NOS: 19 and 23-25.

*(A6) Treatment of influenza virus infection.* Another class of exemplary antisense antiviral compounds are used in inhibition of growth of viruses of the Orthomyxoviridae family and in the treatment of a viral infection. The host cell is contacted with an antisense oligomer conjugated to a lung-specific carrier peptide, as described herein, and containing a base sequence effective to hybridize to a target region selected from the following: i) the 5' or 3' terminal 25 bases of a negative sense viral RNA segment of Influenzavirus A, Influenzavirus B and Influenzavirus C, ii) the terminal 30 bases of the 3' terminus of a positive sense cRNA of Influenzavirus A, Influenzavirus B and Influenzavirus C, and iii) the 50 bases surrounding the AUG start codon of an influenza viral mRNA. (See, *e.g*., PCT Pubn. No. WO/2006/047683 or U.S. Appn. Pubn. No. 20070004661.)

The compounds are particularly useful in the treatment of influenza virus infection in a mammal. The carrier peptide-oligomer conjugate may be administered to a mammalian subject infected with the influenza virus, or at risk of infection with the influenza virus.

Exemplary antisense oligomers that target the influenza A virus are listed as SEQ ID NOs: 41 and 42. These sequences will target most, if not all, influenza A virus strains because of the high degree of homology between strains at the respective targets. Exemplary lung-specific carrier peptide for conjugating to these antisense oligomers are the B (CP06062), P007 and (RB)₈ peptides (SEQ ID NOS: 19, 11 and 14, respectively).

*(A7) Inhibition of inflammatory arthritis induced by TNF-α*. In another embodiment, the expression of the TNF receptor (TNFR2) can be altered with antisense oligomers conjugated to liver-specific carrier peptide, as described in the present invention, to induce the expression of an alternatively spliced soluble TNF-α receptor 2 isoform (sTNFR2). This naturally occurring alternatively spliced isoform of the TNFR2 gene provides antiinflammatory properties because it antagonizes TNF-α biological activity. Overexpression of the sTNFR2 isoform using antisense oligomers conjugated to liver specific carrier peptides and targeted to the exon 7 splice acceptor region of the human TNFR2 gene (e.g., SEQ ID NO: 33) provides an immunotherapeutic approach to inhibit inflammatory arthritis, specifically arthritis induced by TNF-α. Exemplary carrier peptides are the B (CP06062), G, H and I peptides (SEQ ID NOs: 19 and 23-25).

*(A8) Modulation of immunoregilatory function, including treatment of immune system disorders.* The use of antisense oligomers for treating various immune-related conditions has been described. For example, administration of an antisense oligomer spanning the splice junction between intron 1 and exon 2 of preprocessed T cell antigen-4 (CTLA-4) mRNA results in an increased ratio of processed mRNA encoding ligand-independent CTLA-4 to processed mRNA encoding full-length CTLA-4, which is useful for suppressing an immune response in a mammalian subject, e.g. for the treatment or prevention of an autoimmune condition or transplantation rejection. See co-owned U.S. Appn. Pubn. No. 20070111962.

In other applications, an antisense oligomer targeted against cFLIP causes activation induced cell death (AICD) of activated lymphocytes, as described in co-owned U.S. Appn. Pubn. No. 20050203041. Antisense targeted to IL-10 is effective for reversal of IL-10-induced immunosuppression, as described in co-owned provisional application USSN 60/009,464.

Effectiveness of any of these oligomers can be enhanced by conjugation to a peptide having the sequence represented by SEQ ID NO: 27, which is selective for delivery to bone marrow.

### B. Combination with Homing Peptides

The cell-penetrating peptides (CPPs) of the invention can be used in conjunction with homing peptides selective for the target tissue, to further enhance tissue-specific delivery. Isolation of organ homing peptides can be accomplished using a variety of techniques, including combinatorial phage display libraries, as described by Kolonin *et al.* (Kolonin, Sun *et al.* 2006). Techniques for isolation of organ homing peptides are also described by the same researchers in U.S. Appn. Pubn. No. 20040170955 and by Vodyanoy et al. in U.S. Appn. Pubn. No. 20030640466. These homing peptides bind to tissue-specific receptors based on the similarity of the selected peptide to the receptor's natural ligand.

An example of the utility of this approach can be found in the application of muscle-binding peptides (Samoylova and Smith, 1999; Vodyanoy et al., U.S. Appn. Pubn. No. 20030640466) coupled to antisense oligomers designed to be therapeutic treatments for Duchenne muscular dystrophy (DMD) (Gebski, Mann *et al.* 2003; Alter, Lou *et al*. 2006) (PCT Pubn. No. WO2006000057). The heptapeptide sequence ASSLNIA has enhanced *in vivo* skeletal and cardiac muscle binding properties, as described by Samoylova and Smith. As a further example, a pancreas-homing peptide, CRVASVLPC, mimics the natural prolactin receptor ligand (Kolonin, Sun *et al.* 2006).

Coupling tissue specific homing peptides with the cell penetrating peptides of the present invention provides enhanced tissue specific delivery of antisense PMO oligomers. An exemplary dual peptide molecule has a cell penetrating peptide to one terminus, e.g. at the 5' end of the antisense oligomer, as described herein, and a homing peptide coupled to the other terminus, i.e. the 3' terminus. The homing peptide localizes the peptide-conjugated PMO to the target tissue, where the cell-penetrating peptide moiety effects transport into the cells of the tissue.

Alternatively, a preferred exemplary dual peptide molecule would have both a homing peptide (HP) and cell-penetrating peptide (CPP) conjugated to one end, e.g. the 5' terminus of the antisense oligomer, in either a HP-CPP-PMO configuration or, more preferably, a CPP-HP-PMO configuration.

For example, a PMO designed to induce therapeutic exon skipping of the dystrophin gene, as described by Wilton et al. (PCT Publication WO2006/000057), conjugated at the 3' terminus to the muscle-binding peptide ASSLNIA, and further coupled at the 5' terminus to a cell penetrating peptide of the present invention, preferably having enhanced selectivity for muscle tissue, will provide enhanced therapeutic potential in the treatment of DMD. This is exemplified in Example 2, below.

Similarly, the pancreas-specific homing peptide described above, CRVASVLPC, could be coupled to the 3' end of a PMO, and a CPP of the present invention, preferably having enhanced selectivity for pancrease, could be coupled to the 5' terminus. The pancreatic homing peptide would localize the conjugate to the pancreas, and the CPP would then deliver the conjugate internally to the cells of the pancreas. Alternatively, and preferably, both the pancreas-specific homing peptide and the CPP could be coupled to the 5' terminus of the antisense oligomer, in either the HP-CPP-PMO or CPP-HP-PMO configuration.

Examples of homing peptides known to the art are listed below in Table 2 along with their target tissues. Any of these homing peptides can be coupled to an appropriate tissue-specific CPP of the present invention to further enhance tissue-specific delivery of antisense oligomers.

**Table 3. Examples of Tissue-specific Homing Peptides (HP)**

| **Target Tissue** | **Peptide Sequence (NH₂ to COOH)** | **SEQ ID NO.** |
|---|---|---|
| Skeletal Muscle - SMP1 | ASSLNIA | 51 |
| SMP2 | SLGSFP | 52 |
| SMP3 | SGASAV | 53 |
| SMP4 | GRSGAR | 54 |
| SMP5 | TARGEHKEEELI | 55 |
| Cardiac Muscle - CMP1 | WLSEAGPWTVRALRGTGSW | 56 |
| CMP2 | VTVRALRGTSW | 57 |
| CMP3 | VVTVRALRGTGSW | 58 |
| CMP4 | CRPPR | 59 |
| CMP5 | SKTFNTHPQSTP | 60 |
| Lung | CGFECVRQCPERC | 61 |
| Prostate | SMAIARL | 62 |
| Brain | CLSSRLDAC | 63 |
| Hematopoietic stem cells | STFTKSP | 64 |
| Skin | CVALCREACGEGC | 65 |
| Pancreas - Panc1 | SWCEPGWCR | 66 |
| Panc2 | CRVASVLPC | 67 |
| Panc3 | LSGTPERSGQA VKVKLKAIP | 68 |
| Intestine | YSGKWGW | 69 |
| Bladder tumor | CSNRDARRC | 70 |
| Breast tumor | cyclic peptide cCPGPEGAGC (PEGA) | 71 |
| Dendritic cells | FYPSYHSTPQRP | 72 |
| Tumor | CGKRK | 73 |
| Vascular tumors | KDEPQRRSAR LSAKPAPPKP EPKPKKAPAK K | 74 |
| Endothelial tumors | CGNKRTRGC | 75 |
| Hepatocytes | FQHPSFI | 76 |

### C. Delivery of siRNA

The CPP of the present invention can also be used to deliver siRNA molecules. It is known in the art that the introduction of small interfering RNA duplexes (siRNA) into the cytoplasm of mammalian cells triggers an evolutionarily conserved process catalyzing the specific downregulation of mRNA targets through siRNA oligonucleotide complementation and mRNA cleavage (Sontheimer 2005). The potential for siRNA in treating multiple disease states has become the focus of a large number of academic laboratories and pharmaceutical companies around the world (Behlke 2006). There is significant potential for the CPP of the present invention to deliver siRNAs, thereby bypassing the multiple *in vivo* complications shown for the methodologies currently utilized for nucleic acid delivery.

However, in order to avoid neutralizing the cationic charges of the CPPs (which are believed to play a significant role in cellular internalization), it is preferred to employ dsRNA binding proteins (DRBP). DRBPs bind siRNAs in a sequence-independent manner and provide a means to mask the negative charge on siRNA oligonucleotides, thereby allowing for efficient CPP-mediated siRNA delivery.

### IX. Peptide-Antisense Oligomer Conjugate Compositions

### A. Conjugates for Specific Applications

Therapeutic conjugates comprising selected transport peptide sequences are also provided by the invention. These include conjugates comprising a carrier peptide as described herein, preferably selected from the group consisting of SEQ ID NOs: 20, 21, 23, 24, 25, and 27, conjugated, via a terminus of the peptide, to a therapeutic compound. In one embodiment, the compound is a nucleic acid analog, such as a PMO; in other embodiments, the compound is a non-nucleic acid compound, such as a small organic compound.

The conjugates may further comprise a targeting moiety effective to bind to tissue specific receptors of a target tissue type, linked to the therapeutic compound or, preferably, to another terminus of the carrier peptide. In particularly preferred embodiments, a homing peptide such as described above is conjugated to therapeutic compound or to the cell-penetrating peptide.

Of particular interest are specific conjugates as described below.

The invention provides a conjugate for use in treating prostate cancer in a mammalian subject, comprising a carrier peptide having the sequence identified as SEQ ID NO: 23, and conjugated to a terminus of the peptide, an antisense oligomer targeted against human androgen receptor protein, such as a PMO having SEQ ID NO: 43.

Also provided is a peptide conjugate compound for use in for use in treating polycystic kidney disease prostate cancer in a mammalian subject, comprising a carrier peptide having a sequence selected from the SEQ ID NOs: 13, 14, 21, and 27, and particularly SEQ ID NO: 13; and conjugated to a terminus of the peptide, an antisense oligomer targeted against human c-myc protein, such as a PMO having a sequence selected from SEQ ID NO: 31-33.

Also provided is a peptide conjugate compound for enhancing stem cell proliferation and survival in peripheral blood, comprising a carrier peptide having a sequence selected from the group consisting of SEQ ID NOs: 11, 14, 19 and 27, and conjugated to a terminus of the peptide, an antisense oligomer targeted against human TGF-β, such as a PMO having a sequence selected from SEQ ID NOs: 44-46.

Also provided is a peptide conjugate compound for use in treating Duchenne muscular dystrophy, comprising a carrier peptide having a sequence selected from the group consisting of SEQ ID NOs: 6, 13, 19, and 20, and conjugated to a terminus of the peptide, an antisense oligonucleotide capable of producing exon skipping in the DMD protein, such as a PMO having SEQ ID NO: 44, to restore partial activity of the DMD protein.

Also provided is a peptide conjugate compound for use in treating or reducing the risk of restenosis in a blood vessel, comprising a carrier peptide is selected from the group consisting of SEQ ID NOs: 19 and 21, and conjugated to a terminus of the peptide, an antisense oligomer targeted against human c-myc, such as a PMO having a sequence selected from SEQ ID NOs: 31-33.

Also provided is a peptide conjugate compound for use in treating a respiratory viral infection, comprising a carrier peptide having the sequence identified by SEQ ID NO: 10, and conjugated to a terminus of the peptide, an antisense oligomer targeted against influenza A virus, such as a PMO having a sequence selected from SEQ ID NOs: 41 and 42, or against respiratory syncytial virus, such as a PMO having a sequence identified by SEQ ID NO: 48.

Also provided is a peptide conjugate compound for use in treating a respiratory bacterial infection, comprising a carrier peptide having the sequence identified by SEQ ID NO: 10, and conjugated to a terminus of the peptide, an antisense oligomer targeted against a bacterial 16S rRNA, such as a PMO having SEQ ID NO: 47.

Also provided is a peptide conjugate compound for use in metabolic redirection of a xenobiotic compound normally metabolized in the liver, comprising a carrier peptide having a sequence selected from the group consisting of SEQ ID NOS: 19, 23, 24 and 25; and conjugated to a terminus of the peptide, antisense oligomer targeted against a liver P450 enzyme, such as a PMO having a sequence selected from SEQ ID NOs: 28-30.

Also provided is a peptide conjugate compound for use in treating viral hepatitis, comprising a carrier peptide having a sequence selected from the group consisting of SEQ ID NOS: 19, 23, 24 and 25; and conjugated to a terminus of the peptide, an antisense oligomer targeted against HCV start region or IRES, such as a PMO having SEQ ID NO: 39 or 40.

### B. Morpholino Oligomers having Cationic Intersubunit Linkages

In preferred embodiments, as noted above, the antisense oligomer is a phosphorodiamidate morpholino oligonucleotide (PMO). The PMO may include between about 20-50% positively charged backbone linkages, as described below and further in PCT Pubn. No. WO 2008036127.

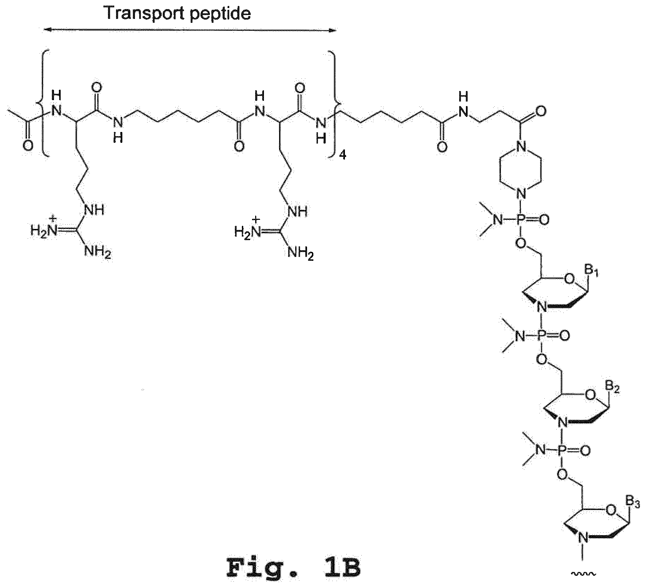

The cationic PMOs (PMO+) are morpholino oligomers in which at least one intersubunit linkage between two consecutive morpholino ring structures contains a pendant cationic group. The pendant group bears a distal nitrogen atom that can bear a positive charge at neutral or near-neutral (e.g. physiological) pH. Examples are shown in Figs. 1B-C.

The intersubunit linkages in these oligomers are preferably phosphorus-containing linkages, having the structure: 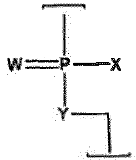 where
W is S or O, and is preferably O,
X = NR¹R² or OR⁶,
Y = O or NR⁷,
   and each said linkage in the oligomer is selected from:
      (a)uncharged linkage (a), where each of R¹, R², R⁶ and R⁷ is independently selected from hydrogen and lower alkyl;
      (**b1**) cationic linkage (b1), where X = NR¹R² and Y = O, and NR¹R² represents an optionally substituted piperazino group, such that R¹R² = -CHRCHRN(R³)(R⁴)CHRCHR-, where
   each R is independently H or CH₃,
   R⁴ is H, CH₃, or an electron pair, and
   R³ is selected from H, lower alkyl, e.g. CH₃, C(=NH)NH₂, Z-L-NHC(=NH)NH₂, and {C(O)CHR'NH}ₘH, where: Z is C(O) or a direct bond, L is an optional linker up to 18 atoms in length, preferably up to 12 atoms, and more preferably up to 8 atoms in length, having bonds selected from alkyl, alkoxy, and alkylamino, R' is a side chain of a naturally occurring amino acid or a one- or two-carbon homolog thereof, and m is 1 to 6, preferably 1 to 4;
      (**b2**) cationic linkage (b2), where X = NR¹R² and Y = O, R¹ = H or CH₃, and R² = LNR³R⁴R⁵, where L, R³, and R⁴ are as defined above, and R⁵ is H, lower alkyl, or lower (alkoxy)alkyl; and
      (**b3**) cationic linkage (b3), where Y = NR⁷ and X = OR⁶, and R⁷ = LNR³R⁴R⁵, where L, R³, R⁴ and R⁵ are as defined above, and R⁶ is H or lower alkyl;
   and at least one said linkage is selected from cationic linkages (b1), (b2), and (b3).

Preferably, the oligomer includes at least two consecutive linkages of type (a) (i.e. uncharged linkages). In further embodiments, at least 5% of the linkages in the oligomer are cationic linkages (i.e. type (b1), (b2), or (b3)); for example, 10% to 80%, 10% to 50%, or 10% to 35% of the linkages may be cationic linkages.

In one embodiment, at least one linkage is of type (b1), where, preferably, each R is H, R⁴ is H, CH₃, or an electron pair, and R³ is selected from H, lower alkyl, e.g. CH₃, C(=NH)NH₂, and C(O)-L-NHC(=NH)NH₂ The latter two embodiments of R³ provide a guanidino moiety, either attached directly to the piperazine ring, or pendant to a linker group L, respectively. For ease of synthesis, the variable Z in R³ is preferably C(O) (carbonyl), as shown.

The linker group L, as noted above, contains bonds in its backbone selected from alkyl (e.g. -CH₂-CH₂-), alkoxy (-C-O-), and alkylamino (e.g. -CH₂-NH-), with the proviso that the terminal atoms in L (e.g., those adjacent to carbonyl or nitrogen) are carbon atoms. Although branched linkages (e.g. -CH₂-CHCH₃-) are possible, the linker is preferably unbranched. In one embodiment, the linker is a hydrocarbon linker. Such a linker may have the structure -(CH₂)ₙ-, where n is 1-12, preferably 2-8, and more preferably 2-6.

The use of embodiments of linkage types (b1), (b2) and (b3) above to link morpholino subunits may be illustrated graphically as follows:

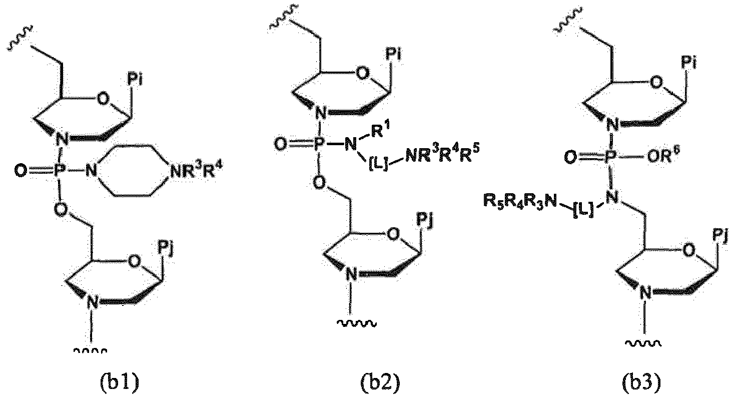

Preferably, all cationic linkages in the oligomer are of the same type; i.e. all of type (b1), all of type (b2), or all of type (b3). The base-pairing moieties Pi may be the same or different, and are generally designed to provide a sequence which binds to a target nucleic acid.

In further embodiments, the cationic linkages are selected from linkages (b1') and (b1") as shown below, where (b1') is referred to herein as a "Pip" linkage and (b1") is referred to herein as a "GuX" linkage:

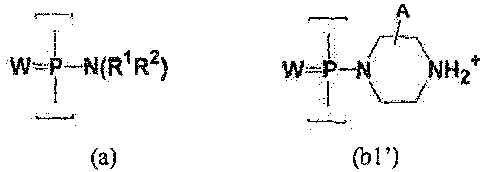

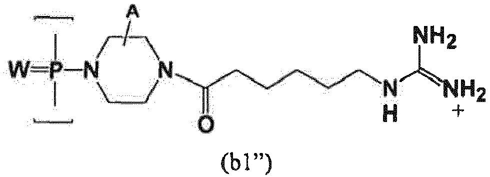

In the structures above, W is S or O, and is preferably O; each of R¹ and R² is independently selected from hydrogen and lower alkyl, and is preferably methyl; and A represents hydrogen or a non-interfering substituent on one or more carbon atoms in (b1') and (b1"). Preferably, the ring carbons in the piperazine ring are unsubstituted; however, they may include non-interfering substituents, such as methyl or fluorine. Preferably, at most one or two carbon atoms is so substituted.

In further embodiments, at least 10% of the linkages are of type (b1') or (b1"); for example, 20% to 80%, 20% to 50%, or 20% to 30% of the linkages may be of type (b1') or (b1").

In other embodiments, the oligomer contains no linkages of the type (b1') above. Alternatively, the oligomer contains no linkages of type (b1) where each R is H, R³ is H or CH₃, and R⁴ is H, CH₃, or an electron pair.

Oligomers having any number of cationic linkages can be used, including fully cationic-linked oligomers. Preferably, however, the oligomers are partially charged, having, for example, 5, 10, 20, 30, 40, 50, 60, 70, 80 or 90 percent cationic linkages. In selected embodiments, about 10 to 80, 20 to 80, 20 to 60, 20 to 50, 20 to 40, or about 20 to 35 percent of the linkages are cationic.

In one embodiment, the cationic linkages are interspersed along the backbone. The partially charged oligomers preferably contain at least two consecutive uncharged linkages; that is, the oligomer preferably does not have a strictly alternating pattern along its entire length.

Also considered are oligomers having blocks of cationic linkages and blocks of uncharged linkages; for example, a central block of uncharged linkages may be flanked by blocks of cationic linkages, or vice versa. In one embodiment, the oligomer has approximately equal-length 5", 3" and center regions, and the percentage of cationic linkages in the center region is greater than about 50%, preferably greater than about 70%.

Oligomers for use in antisense applications generally range in length from about 10 to about 40 subunits, more preferably about 15 to 25 subunits. For example, a cationic oligomer having 19-20 subunits, a useful length for an antisense oligomer, may ideally have two to seven, e.g. four to six, or three to five, cationic linkages, and the remainder uncharged linkages. An oligomer having 14-15 subunits may ideally have two to five, e.g. 3 or 4, cationic linkages and the remainder uncharged linkages.

Each morpholino ring structure supports a base pairing moiety, to form a sequence of base pairing moieties which is typically designed to hybridize to a selected antisense target in a cell or in a subject being treated. The base pairing moiety may be a purine or pyrimidine found in native DNA or RNA (A, G, C, T, or U) or an analog, such as hypoxanthine (the base component of the nucleoside inosine) or 5-methyl cytosine.

As noted above, the substantially uncharged oligonucleotide may be modified to include one or more charged linkages, *e.g*. up to about 1 per every 2-5 uncharged linkages, typically 3-5 per every 10 uncharged linkages. Optimal improvement in antisense activity is seen where up to about half of the backbone linkages are cationic. Some, but not maximum enhancement is typically seen with a small number e.g., 10-20% cationic linkages; where the number of cationic linkages exceeds 50-60%, the sequence specificity of the antisense binding to its target may be compromised or lost.

The enhancement seen with added cationic backbone charges may, in some case, be further enhanced by distributing the bulk of the charges close of the "center-region" backbone linkages of the antisense oligonucleotide, e.g., in a 20-mer oligonucleotide with 8 cationic backbone linkages, having 70%-100% of these charged linkages localized in the 10 centermost linkages.

### C. Other Oligomer Types

Delivery of alternative antisense chemistries can also benefit from the disclosed carrier peptide. Specific examples of other antisense compounds useful in this invention include those in which at least one, or all, of the internucleotide bridging phosphate residues are modified phosphates, such as methyl phosphonates, phosphorothioates, or phosphoramidates. Also included are molecules wherein at least one, or all, of the nucleotides contains a 2' lower alkyl moiety (e.g., C1-C4, linear or branched, saturated or unsaturated alkyl, such as methyl, ethyl, ethenyl, propyl, 1-propenyl, 2-propenyl, or isopropyl).

In other oligonucleotide mimetics, both the sugar and the internucleoside linkage, i.e., the backbone, of the nucleotide units are modified. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-phosphate backbone of an oligonucleotide is replaced with an amide containing backbone, in particular an aminoethylglycine backbone.

Modified oligonucleotides may be classified as "chimeric", e.g. containing at least one region wherein the oligonucleotide is modified so as to confer increased resistance to nuclease degradation or increased cellular uptake, and an additional region for increased binding affinity for the target nucleic acid.

### EXAMPLES

The following examples are intended to illustrate but not to limit the invention.

### Materials and Methods

### In Vitro and In Vivo Assays

Nuclear Activity Assay. The effectiveness of each P-PMO conjugate was determined in a splice-correction assay to assess nuclear activity which utilizes a P-PMO targeted splice site in a plasmid created by an interruption in the luciferase coding sequence by the human β-globin thalassemic intron 2 which carries a mutated splice site at nucleotide 705 (pLuc705). The plasmid is stably transfected in HeLa S3 cells, allowing for easy comparison of the relative efficiency of various carrier peptides to deliver PMO (705; 5'-CCT CTT ACC TCA GTT ACA-3'; SEQ ID NO: 1) capable of restoring splice-correction in cell nuclei. Cells were cultured in RPMI 1640 medium supplemented with 2 mM L-Glutamine, 100 U/mL penicillin, and 10% fetal bovine serum (FBS) at 37°C in a humidified atmosphere containing 5% CO₂, and seeded for 20 hours prior to 2 µM P-PMO treatment. All cell treatments with P-PMO were carried out in OptiMEM medium with or without FBS for 24 hours. After cell treatment, restoration of correct splice-correction was measured by positive readout of luciferase expression in cell lysates on an Flx 800 microplate fluorescence-luminescence reader with excitation at 485 nm and emission at 524 nm.

Cell Uptake Assay. The cellular uptake of P-PMO in HeLa pLuc705 cells was determined using 3'-carboxyfluorescein-tagged P-PMO (P-PMOF) and flow cytometry. Cells were seeded for 20 hours prior to 2 µM P-PMOF treatment. After treatment, cells were trypsinized to remove any cell membrane-bound P-PMOF, and washed and resuspended in PBS (Hyclone, Ogden, UT) containing 1% FBS and 0.2% NaN₃. Cell uptake of P-PMOF was then determined by flow cytometry on a FC-500 Beckman Coulter (Fullerton, CA) cytometer and data was processed using FCS Express 2 software (De Novo Software, Thornhill, Ontario, Canada).

RNA Extraction. Tissue RNA was extracted using Qiagen's RNeasy Mini Kit (Qiagen USA, Valencia, CA) per manufacturer's protocol. All isolated RNA was stored at -80°C. RT-PCR. Restoration of splice-correction was determined by RT-PCR amplification of EGFP mRNA extracted from tissues harvested from P-PMO treated EGFP-654 transgenic mice using the Invitrogen SuperScript™ III One-Step RT-PCR System.

### Toxicity Assays

The cellular toxicity of P-PMOs was determined by methylthiazoletetrazolium-survival (MTT), propidium iodine (PI) exclusion, and hemolysis assays, which measured the effects of the P-PMOs on cellular metabolic activity, membrane integrity, and red blood cell compatibility, respectively.

MTT Analysis. For MTT analysis, cells were seeded at a concentration of 9000 cells/well in 96 well plates for 20 hours then treated with P-PMO ranging in concentration from 2-60 µM. MTT solution was then added to the cells for 4 hours and cellular metabolic activity was measured by reading the absorbance of the treatment medium and normalizing the absorbance of the P-PMO treated samples to the absorbance mean of untreated samples. Microscopic images of P-PMO treated cells were visualized on a Nikon Diaphot inverted microscope (Melville, NY) and processed by Magnafire software (Optronics, Goleta, CA) for correlation with MTT results. All assays were done using HeLa pLuc705 cells. Microscopic images of P-PMO treated cells were visualized on a Nikon Diaphot inverted microscope (Melville, NY) and processed by Magnafire software (Optronics, Goleta, CA) for correlation with MTT results. All assays were done using HeLa pLuc705 cells.

Propidium Iodine-Exclusion. For PI-exclusion analysis, cells were seeded at a concentration of 100,000 cells/well in 12-well plates for 20 hours then treated with P-PMO ranging in concentration from 2-60 µM. Cells were then trypsinized, washed in PBS, and resuspended in PBS containing PI for 15 minutes. Detection of unhealthy or damaged cellular membranes was done by analyzing cells for PI uptake by flow cytometry.

Red Blood Cell Compatibility. Hemolytic activities in red blood cells exposed to P-PMO ranging in concentration from 2-60 µM was determined using fresh rat blood according to an established method (Fischer, Li *et al*. 2003).

MDX Mouse Experiments. Experiments using the MDX mouse strain were performed essentially as described by Jearawiriyapaisarn, Moulton *et al*., 2008.

### Example 1. Evaluation of Cell Penetrating Peptide Conjugated PMOs in the EGFP-654 Transgenic Mouse Model

A PMO (designated 654; 5'-GCT ATT ACC TTA ACC CAG-3'; SEQ ID NO: 2) designed to restore correct splicing in the enhanced green fluorescent protein (EGFP) gene was conjugated to various cell penetrating peptides (SEQ ID NOS: 2, 3, 6, 11, 13-14, 19, 20-27) to produce P-PMOs (peptide-conjugated PMOs), which were evaluated *in vivo* for their splice-correction activity and toxicity in the EGFP-654 transgenic mouse model (Sazani, Gemignani *et al*. 2002). In this model, the EGFP-654 gene encoding for functional EGFP is interrupted by an aberrantly-spliced mutated intron, and cellular uptake of EGFP-654 targeted P-PMOs can be evaluated by RT-PCR detection of the restored EGFP-654 splice product in tissues.

Female EGFP-654 transgenic mice were injected IP once daily for 4 consecutive days with saline or a 12.5 mg/kg dose of P-PMO. Post treatment on day 4, the heart, muscles, liver, kidney, lungs, small intestine, colon, stomach, mammary gland, thymus, spleen, ovary, skin, bone marrow, and brain were harvested, and extracted RNA was evaluated by RT-PCR and densitometry of PCR products to determine % correction. Toxicity of P-PMOs was evaluated by measurement of mouse weights over the course of treatments and immediately prior to necropsy.

Restoration of functional EGFP splice products post treatment with various P-PMOs based on RT-PCR analysis of tissues is shown in Figures 7A-P. Analysis of toxicity based on weights from P-PMO treated mice indicated minimal toxicity (not shown). Optimal carrier peptide uptake for each tissue (indicated by a *) based on these results is summarized in Table 2 (see above).

### Example 2. Evaluation of PMOs Conjugated to a Cell Penetrating Peptide (CPP) and/or a Muscle Specific Homing Peptide (HP) in the MDX Murine Model of Duschenes Muscular Dystrophy

MDX mice were treated with a series of P-PMO (peptide-conjugated PMOs) containing various combinations of muscle-specific CPPs and HPs conjugated to the M23d antisense PMO. The muscle specific CPP used was the "B peptide", also designated CP06062 (SEQ ID NO: 19), and the muscle specific homing peptide, designated SMP1, was SEQ ID NO: 51. Four combinations were tested including CP06062-PMO, MSP-PMO, CP06062-MSP-PMO and MSP-CP06062-PMO, whose compositions are shown in the appended Sequence Table. The M23d antisense PMO (SEQ ID NO: 77) has a sequence targeted to induce an exon 23 skip in the murine dystrophin gene and restores functional dystrophin.

The mice received six weekly intravenous injections of a 3mg/kg dose. The treated mice were sacrificed and various muscle tissues were removed and stained for full-length dystrophin using a dystrophin-specific fluorescent antibody stain.

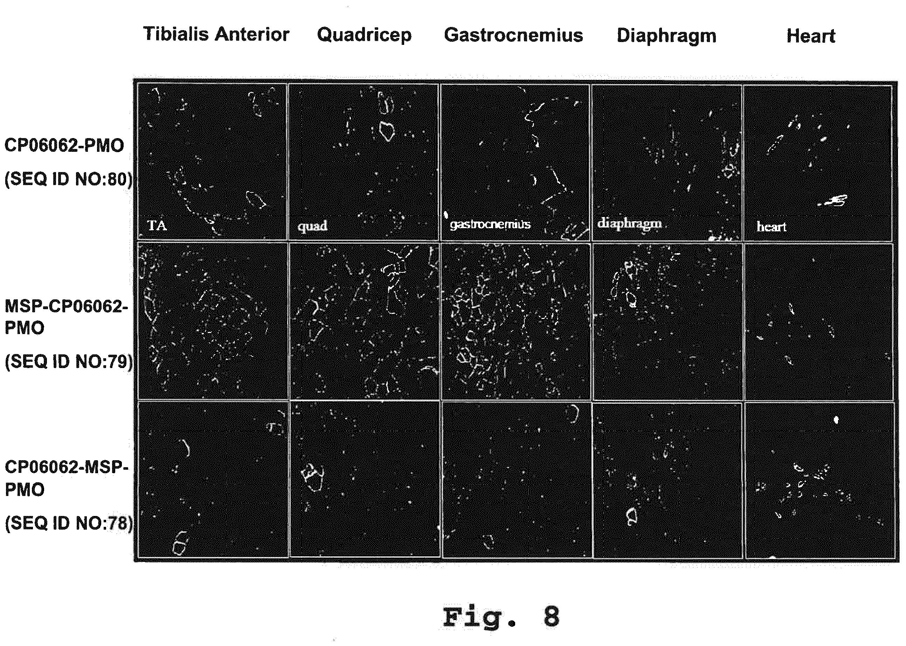

The results for the CP06062-PMO, MSP-CP06062-PMO and CP06062-MSP-PMO conjugates in five different muscle tissues are shown in Figure 8. As can be seen, the dystrophin-specific stain is in much greater evidence for the MSP-CP06062-PMO compound than for the other two conjugates, with the exception of heart muscle, where the CP06062-MSP-PMO conjugate appeared to have the greatest activity. The observation that the CP06062-MSP-PMO compound was more effective than the CP06062-PMO conjugate was confirmed by immunoblot and PCR assays (data not shown). In separate experiments (data not shown), an MSP-PMO conjugate induced full-length dystrophin at a level less than the CP06062-PMO conjugate.

Additional examples of muscle-specific delivery of the CP06062-M23d conjugate to tissues of the MDX mouse can be found in Jearawiriyapaisarn, Moulton *et al*., 2008, cited above.

In summary, the combination of the muscle specific homing peptide and muscle specific cell penetrating peptide significantly improved the delivery of the M23d antisense peptide as measured in this *in vivo* system. The MSP-CP06062-PMO ordering of the peptide moieties was observed to induce the highest level of full-length dystrophin and is a preferred embodiment.

### Sequence Table

| **Designation(s)** | **Sequence** | **SEQ ID N9.^{a}** |
|---|---|---|
| **Antisense Oligomers** | | |
| 705 | 5'-CCT CTT ACC TCA GTT ACA-3' | 1 |
| 654 | 5'-GCT ATT ACC TTA ACC CAG-3' | 2 |

| **Cell-Penetrating Peptides (CPP)** | | |
|---|---|---|
| R₈ | RRRRRRRR-XB | 3 |
| r₈ | rrrrrrrr-XB | 4 |
| R₉ | RRRRRRRRR-XB | 5 |
| (RX)₈ | RXRXRXRXRXRXRXRX-B | 6 |
| (rX)₈ | rXrXrXrXrXrXrXrX-B | 7 |
| (RX)₇ | RXRXRXRXRXRXRX-B | 8 |
| (RX)₈ | RXRXRXRXRX-B | 9 |
| (RX)₃ | RXRXRX-B | 10 |
| (RXR)₄ | RXRRXRRXRRXRX-B | 11 |
| (rXR)₄ | rXRrXRrXRrXR-B | 12 |
| (rXr)₄ | rXrrXrrXrrXr-XB | 13 |
| (RB)₈ | RBRBRBRBRBRBRBRB-B | 14 |
| (rB)₈ | rBrBrBrBrBrBrBrB-B | 15 |
| (RB)₇ | RBRBRBRBRBRBRB-B | 16 |
| (RB)₅ | RBRBRBRBRB-B | 17 |
| (RB)₃ | RBRBRB-B | 18 |
| B(3b); CP06062; (RXRRBR)₂-XB | RXRRBRRXRRBR-XB | 19 |
| D(2); (RB)₅RXRBRX-B | RBRBRBRBRBRXRBRX-B | 20 |
| E(3c); (RBRBRBRX)₂-X | RBRBRBRXRBRBRBRX-X | 21 |
| F(3a); X-RB)₃RX(RB)₃RX | X-RBRBRBRXRBRBRBRX | 22 |
| G(4b); (RBRX)₄B | RBRXRBRXRBRXRBRX-B | 23 |
| H(4a); (RB)₄(RX)₄B | RBRBRBRBRXRXRXRX-B | 24 |
| I(3d); RX(RB)₂RX(RB)₃RX-X | RXRBRBRXRBRBRBRX-X | 25 |
| C(4c); (RXR)₃RBR-XB | RXRRXRRXRRBR-XB | 26 |
| (RB)₇RX-B | RBRBRBRBRBRBRBRX-B | 27 |

| **Homing peptides (HP) (NH₂ to COOH)** | | |
|---|---|---|
| Skeletal Muscle - SMP1 | ASSLNIA | 51 |
| SMP2 | SLGSFP | 52 |
| SMP3 | SGASAV | 53 |
| SMP4 | GRSGAR | 54 |
| SMP5 | TARGEHKEEELI | 55 |
| Cardiac Muscle - CMP1 | WLSEAGPWTVRALRGTGSW | 56 |
| CMP2 | VTVRALRGTSW | 57 |
| CMP3 | VVTVRALRGTGSW | 58 |
| CMP4 | CRPPR | 59 |
| CMP5 | SKTFNTHPQSTP | 60 |
| Lung | CGFECVRQCPERC | 61 |
| Prostate | SMAIARL | 62 |
| Brain | CLSSRLDAC | 63 |
| Hematopoietic stem cells | STFTKSP | 64 |
| Skin | CVALCREACGEGC | 65 |
| Pancreas - Panel | SWCEPGWCR | 66 |
| Panc2 | CRVASVLPC | 67 |
| Panc3 | LSGTPERSGQAVKVKLKAIP | 68 |
| Intestine | YSGKWGW | 69 |
| Bladder tumor | CSNRDARRC | 70 |
| Breast tumor | cyclic peptide cCPGPEGAGC(PEGA) | 71 |
| Dendritic cells | FYPSYHSTPQRP | 72 |
| Tumor | CGKRK | 73 |
| Vascular tumors | KDEPQRRSARLSAKPAPPKPEPKPKK APAKK | 74 |
| Endothelial tumors | CGNKRTRGC | 75 |
| Hepatocytes | FQHPSFI | 76 |

| **Antisense Oligomers and Target Sequences (5' to 3')** | | |
|---|---|---|
| CYP3A4 | GTCTGGGATGAGAGCCATCAC | 28 |
| CYP3A4 | CTGGGATGAGAGCCATCAC | 29 |
| CYP3A4 | CTGGGATGAGAGCCATCACT | 30 |
| hu c-myc (AVI-4126) | ACGTTGAGGGGCATCGTCGC | 31 |
| hu c-myc | GGCATCGTCGCGGGAGGCTC | 32 |
| Hu-TNFR2 | CCACAATCAGTCCTAG | 33 |
| AVI-4658 (hu-dystrophin, exon 51) | CTCCAACATCAAGGAAGATGGCAT TTCTAG | 34 |
| Human Myostatin SD1 | ACTCTGTAGGCATGGTAATG | 35 |
| Human Myostatin SD2 | CAGCCCATCTTCTCCTGG | 36 |
| Human Myostatin SA2 | CACTTGCATTAGAAAATCAG | 37 |
| Human Myostatin SA3 | CTTGACCTCTAAAAACGGATT | 38 |
| Human Mysostatin-AUG | GAGTTGCAGTTTTTGCATG | 39 |
| HCV-AUG | GTGCTCATGGTGCACGGTC | 40 |
| FLUA (-)NP-3'trm | AGCAAAAGCAGGGTAGATAATC | 41 |
| FLUA PB1-AUG | GACATCCATTCAAATGGTTTG | 42 |
| Human androgen receptor | CTGCACTTCCATCCTTGAGC | 43 |
| TGF-β1 | GAGGGCGGCATGGGGGAGGC | 44 |
| TGF-β Type II receptor | GACCCATGGCAGCCCCCGTCG | 45 |
| TGF-β1 splice junction | GCAGCAGTTCTTCTCCGTGG | 46 |
| Bacterial 16S rRNA consensus | GGACTACGACGCACTTTATGAG | 47 |
| Respiratory syncytial virus | TAATGGGATCCATTTTGTCCC | 48 |
| AVI-5656 (hu-dystrophin, exon 50) | CCACTCAGAGCTCAGATCTTCTAAC TTCC | 49 |
| HCV-143' | GTACTCACCGGTTCCGCAGACCAC | 50 |
| M23d antisense PMO | GGCCAAACCTCGGCTTACCTGAAA T | 77 |

| **Cell Penetrating Peptide / Homing Peptide / PMO Conjugates (NH₂ to COOH and 5' to 3')** | | |
|---|---|---|
| **Designation** | **Composition (with linkers)** | **Component SEQ ID Nos.** |
| MSP-PMO | ASSLNIA-XB- GGCCAAACCTCGGCTTACCTGAAAT | 51, 77 |
| CP06062-MSP-PMO | RXRRBRRXRRBR-XB-ASSLNIA-X- GGCCAAACCTCGGCTTACCTGAAAT | 19, 51, 77 |
| MSP-CP06062-PMO | ASSLNIA-X-RXRRBRRXRRBR-B- GGCCAAACCTCGGCTTACCTGAAAT | 51, 19, 77 |
| CP06062-PMO | RXRRBRRXRRBR-XB- GGCCAAACCTCGGCTTACCTGAAAT | 19, 77 |

| | | |
|---|---|---|
| ^{a}In SEQ ID Nos. 3-27, sequences assigned to SEQ ID NO. do not include the linkage portion (X, B, or XB). | | |

The present disclosure will now be further defined in the following paragraphs:
1. A peptide-antisense conjugate for treating a given disease condition, comprising a phosphorodiamidate morpholino antisense oligomer directed against a gene whose expression is associated with the given disease condition in a specific target tissue, and covalently linked thereto, via a linkage X, B, or XB, a cell-penetrating peptide that comprises 8 to 20 amino acid residues and consists of a combination of subsequences (RXR) and (RBR), or a combination of subsequences (RX) and (RB), where R is arginine; B is β-alanine; and each X is independently a neutral linear amino acid -C(O)-(CH₂)ₙ-NH-, where n is 4-6, where the cell-penetrating peptide selectively localizes the antisense oligomer in the target tissue.
2. The conjugate of paragraph 1, wherein n is 5, such that each X is a 6-aminohexanoic acid residue.
3. The conjugate of paragraph 1, wherein the phosphorodiamidate morpholino oligomer contains between about 20-50% positively charged backbone linkages.
4. The conjugate of paragraph 1, for use in treating prostate cancer in a mammalian subject,
   wherein the cell-penetrating peptide has the sequence identified as SEQ ID NO: 23, and the oligomer is targeted against human androgen receptor protein.
5. The conjugate of paragraph 1, for use in treating polycystic kidney disease in a mammalian subject,
   wherein the cell-penetrating peptide is selected from the group consisting of SEQ ID NOs: 13, 14, 21 and 27, and the oligomer is targeted against human c-myc protein.
6. The conjugate of paragraph 1, for enhancing stem cell proliferation and survival in peripheral blood,
   wherein the cell-penetrating peptide having a sequence selected from the group consisting of SEQ ID NOs: 10, 14, 19, and 27, and the oligomer is targeted against human TGF-β.
7. The conjugate of paragraph 1, for use in treating cardiac restenosis,
   wherein the cell-penetrating peptide is selected from the group consisting of SEQ ID NOs: 19 and 21, and the oligomer is targeted against human c-myc.
8. The conjugate of paragraph 1, for use in treating a respiratory viral infection,
   wherein the cell-penetrating peptide has the sequence identified by SEQ ID NO: 10, and
   the oligomer is targeted against influenza A virus or respiratory syncytial virus.
9. The conjugate of paragraph 1, for use in treating a respiratory bacterial infection,
   wherein the cell-penetrating peptide has a sequence selected from the group identified by SEQ ID NO: 13, 14, and 19, and the oligomer is targeted against a bacterial 16S rRNA.
10. The conjugate of paragraph 1, for use in metabolic redirection of a xenobiotic compound normally metabolized in the liver,
   wherein the cell-penetrating peptide has a sequence selected from the group consisting of SEQ ID NOS: 19, 23, 24 and 25; and the oligomer is targeted against a liver P450 enzyme.
11. The conjugate of paragraph 1, for use in treating viral hepatitis,
   wherein the cell-penetrating peptide has a sequence selected from the group consisting of SEQ ID NOS: 19, 23, 24, and 25; and the oligomer is targeted against hepatitis C virus or hepatitis B virus.
12. The conjugate of paragraph 1, for use in treating Duchenne muscular dystrophy,
   wherein the cell-penetrating peptide has a sequence selected from the group consisting of SEQ ID NOs: 6, 13, 19, and 20, and the oligomer has a sequence effective to produce exon skipping in the human dystrophin protein, to restore partial activity of the dystrophin protein.
13. The conjugate of paragraph 1, for use in treating loss of skeletal muscle mass in a human subject, wherein the cell-penetrating peptide has a sequence selected from the group consisting of SEQ ID NOs: 6, 13, 19, and 20, and the antisense oligomer is targeted against human myostatin.
14. The conjugate of paragraph 12 or 13, wherein the cell-penetrating peptide has the sequence SEQ ID NO: 19.
15. The conjugate of paragraph 12 or 13, wherein the antisense oligomer has a sequence selected from the group consisting of SEQ ID NOs: 34, 49, and 81.
16. The conjugate of paragraph 1, further comprising a homing peptide which is selective for a selected target tissue, conjugated to the cell-penetrating peptide, thereby forming a conjugate of the form: cell penetrating peptide - homing peptide - antisense oligomer.
17. The conjugate of paragraph 1, further comprising a homing peptide which is selective for a selected target tissue, conjugated to the cell-penetrating peptide, thereby forming a conjugate of the form: homing peptide - cell penetrating peptide - antisense oligomer.
18. The conjugate of paragraph 12 or 13, further comprising a homing peptide which is selective for muscle tissue, conjugated to the cell-penetrating peptide.
19. The conjugate of paragraph 18, wherein said homing peptide has the sequence identified as SEQ ID NO: 51.
20. The conjugate of paragraph 19, wherein the conjugate is of the form: cell penetrating peptide - homing peptide - antisense oligomer, and has the composition designated herein as CP06062-MSP-PMO.
21. The conjugate of paragraph 19, wherein the conjugate is of the form: homing peptide - cell penetrating peptide - antisense oligomer and has the composition designated herein as MSP- CP06062-PMO.
22. A drug-peptide conjugate for treating a given disease condition, comprising a drug whose action is directed against a specific target tissue, and covalently linked thereto, via a linkage X, B, or XB, a cell-penetrating peptide that comprises 8 to 20 amino acid residues and consists of a combination of subsequences (RXR) and (RBR), or a combination of subsequences (RX) and (RB), where R is arginine; B is β-alanine; and each X is independently a neutral linear amino acid -C(O)-(CH₂)ₙ-NH-, where n is 4-6, where the cell-penetrating peptide selectively localizes the drug in the target tissue.
23. The conjugate of paragraph 22, for use in treating restenosis, wherein
   the cell-penetrating peptide is selected from the group consisting of SEQ ID NOs: 19 and 27, and
   the therapeutic compound is selected from the group consisting of an antisense oligomer targeted against human c-myc, rapamycin, and rapamycin analogs having anti-restenosis activity.
24. The conjugate of paragraph 22, wherein the therapeutic compound is a siRNA.
25. The conjugate of paragraph 24, further comprising a double stranded RNA binding compound to which the siRNA is noncovalently bound.
26. A method for identifying a cell-penetrating peptide useful for targeting a therapeutic compound to a selected mammalian tissue, comprising:
   (a) forming a library of peptide conjugates composed of
      (i) a plurality of different peptides, each 8 to 20 amino acid residues in length and consisting of subsequences selected from the group consisting of RXR, RX, RB, and RBR; where R is arginine, B is β-alanine, and each X is independently -C(O)-(CHR¹)ₙ-NH-, where n is 4-6 and each R' is independently H or methyl, such that at most two R^{1,}s are methyl, and
      (ii) covalently coupled to each peptide via an X, B, or XB linkage, an oligomeric marker compound whose concentration can be assayed in the cells of the selected tissue;
   (b) administering each peptide conjugate to a mammalian subject;
   (c) assaying the level of the marker compound in cells of the selected tissue, after a period sufficient for localization of the administered peptide conjugate in the selected tissue of the mammalian subject; and
   (d) selecting a cell-penetrating peptide useful for targeting a therapeutic compound to the selected mammalian tissue, based on its ability to produce highest or near-highest levels of marker compound, relative to other peptides in the conjugate library, in the selected tissue.
27. The method of paragraph 26, wherein the peptides in the library include at least 8 peptides selected from the group having sequences identified by SEQ ID NOS: 6-27.
28. The method of paragraph 26, wherein X is 6-aminohexanoic acid residue, and the peptide contains at least three X residues and consists of a combination of (RXR) and (RBR) subsequences.
29. The method of paragraph 26, wherein X is 6-aminohexanoic acid residue, and the peptide contains at least three X residues and consists of a combination of (RX) and (RB) subsequences.
30. The method of paragraph 26, for use in preparing a therapeutic conjugate for treating a disease condition associated with said selected tissue in a mammalian subject, the method further comprising:
   selecting a therapeutic compound which is effective against the disease condition when localized in cells of the selected tissue, and
   conjugating the therapeutic compound to one terminus of the selected cell-penetrating peptide.
31. The method of paragraph 27, wherein the therapeutic compound is an oligomeric antisense compound.
32. A cell-penetrating peptide having a sequence selected from the group consisting of SEQ ID NOs: 14-27.
33. A peptide as recited in paragraph 32, having a sequence selected from the group consisting of SEQ ID NOs: 19-27.
34. A peptide as recited in paragraph 33, having a sequence selected from the group consisting of SEQ ID NOs: 19, 23, 24 and 25.
35. A peptide as recited in paragraph 34, having the sequence identified as SEQ ID NO: 19.
36. In a method for preparing an antisense composition useful for treating DMD or a muscle-wasting disease in a mammalian subject, by preparing an antisense oligomer effective to suppress splice-variant truncations in expressed dystrophin protein, or effective to suppress myostatin expression in muscle tissue, respectively, when administered to a mammalian subject, an improvement comprising
   conjugating to the oligomer, a cell-penetrating peptide having the sequence identified as SEQ ID NO: 19.
37. The method of paragraph 36, wherein the improvement further includes conjugating a muscle-homing peptide to the oligomer and cell-penetrating peptide, to form a homing peptide - cell penetrating peptide - antisense oligomer composition.
38. In a method for preparing an antisense composition useful for treating of an inflammatory condition, by preparing an antisense oligomer effective to induce expression of a soluble TNF-α receptor, when the oligomer is administered to a mammalian subject, an improvement comprising
   conjugating to the oligomer, a cell-penetrating peptide having a sequence selected from the group consisting of SEQ ID NOS: 19, 23, 24, and 25.
39. The method of paragraph 38, wherein the improvement further includes conjugating a liver-homing peptide to the oligomer and cell-penetrating peptide, to form a homing peptide - cell penetrating peptide - antisense oligomer composition.
40. In a method for preparing an antisense composition useful for treating an immune condition in a mammalian subject, by preparing an antisense oligomer effective to suppress expression of IL-10, CTLA-4, or cFLIP in leukocytes, when administered to a mammalian subject, an improvement comprising
   conjugating to the oligomer, a cell-penetrating peptide having the sequence identified as SEQ ID NO: 27.
41. The method of paragraph 40, wherein the improvement further includes conjugating a leukocyte-homing peptide to the oligomer and cell-penetrating peptide to form a homing peptide - cell penetrating peptide - antisense oligomer composition.

## Claims

1. A peptide-antisense conjugate, comprising a phosphorodiamidate morpholino antisense oligomer comprising a sequence capable of producing exon skipping in the human DMD protein, to restore partial activity of the protein, and covalently linked thereto, via a linkage comprising X, B, or XB, a cell-penetrating peptide of 8 to 30 amino acid residues comprised of a combination of (RXR) and (RBR) subsequences, or comprised of a combination of (RX) and (RB) subsequences, wherein each R is arginine; each B is β-alanine; and each X is 6-aminohexanoic acid, wherein the cell-penetrating peptide selectively localizes the drug in a target tissue.

2. The peptide-antisense conjugate of claim 1, wherein the cell-penetrating peptide comprises a combination of (RXR) and (RBR) subsequences.

3. The peptide-antisense conjugate of claim 1, wherein the cell-penetrating peptide comprises a combination of (RX) and (RB) subsequences.

4. The peptide-antisense conjugate of claim 1, wherein the cell-penetrating peptide is 8 to 20 amino acid residues.

5. The peptide-antisense conjugate of claim 4, wherein the cell-penetrating peptide comprises a combination of (RXR) and (RBR) subsequences.

6. The peptide-antisense conjugate of claim 4, wherein the cell-penetrating peptide comprises a combination of (RX) and (RB) subsequences.

7. The peptide-antisense conjugate of any one of claims 1-6, further comprising a homing peptide.

8. The peptide-antisense conjugate of claim 7, wherein the homing peptide is a muscle-specific homing peptide.

9. The peptide-antisense conjugate of claim 1, wherein the cell-penetrating peptide comprises a combination of (RXR) and (RBR) subsequences, wherein the conjugate further comprises a muscle-specific homing peptide.

10. The peptide-antisense conjugate of claim 1, wherein the cell-penetrating peptide comprises a combination of (RX) and (RB) subsequences, wherein the conjugate further comprises a muscle-specific homing peptide.

11. A pharmaceutical composition, comprising the peptide-antisense conjugate of any one of claims 1-10 and a pharmaceutically acceptable carrier.
